# NEW EUROPEAN PATENT SPECIFICATION

(11) **EP 2 379 718 B2**
(45) Date of publication and mention of the opposition decision: **30.12.2020**
(45) Mention of the grant of the patent: 20.03.2013
(21) Application number: 09774904.8
(22) Date of filing: 14.12.2009
(51) Int. Cl.: C12N 15/10, C12N 15/81, C40B 40/08

(54) **YEAST DISPLAY SYSTEMS**
HEFE-DISPLAY-SYSTEME
SYSTÈMES DE PRÉSENTATION DE LEVURES

(30) Priority: 16.12.2008 US 122910 P
(43) Date of publication of application: 26.10.2011
(73) Proprietor: Novartis AG, 4056 Basel (CH)
(72) Inventor: LOEW, Andreas, Cambridge, Ma 02139 (US)
(74) Representative: Marshall, Cameron John
(86) International application number: PCT/EP2009/067066
(87) International publication number: WO 2010/069913

(56) References cited:
- WO-A1-99/36569
- WO-A2-2007/046825
- WO-A2-2008/118476
- CEBE REGIS ET AL: "Size of the ligand complex between the N-terminal domain of the gene III coat protein and the non-infectious phage strongly influences the usefulness of in vitro selective infective phage technology" BIOCHEMICAL JOURNAL, THE BIOCHEMICAL SOCIETY, LONDON, GB LNKD- DOI:10.1042/0264-6021:3520841, vol. 352, no. 3, 1 January 2000 (2000-01-01), pages 841-849, XP002211404 ISSN: 0264-6021
- Rakestraw et al: Biotechnol. Prog., vol. 22, 2006, pages 1200-1208,
- Weaver Feldhaus: FEBS Letters, vol. 564, 2004, pages 24-34,
- Boder and Wittrup: Nature Biotechnology, vol. 15, 1997, page 53ff,

## Description

### FIELD

The present invention relates to the field of protein display libraries and library screening. More specifically, the present invention relates to the production of proteins for display on cell surfaces.

### BACKGROUND

Protein binding domains can be predicted from sequence data, however re-designing proteins with improved or altered binding affinities often requires testing of a number of variants of the re-designed protein. Currently the best method for obtaining proteins with desired binding affinities is to generate and screen a protein library including such variants that can include rationally redesigned proteins, randomly altered proteins, or a combination thereof. Libraries of many types of protein, such as immunoglobulins and scaffold proteins and receptors or receptor ligands have successfully been constructed and screened for binding affinity.

There are many methods to screen libraries, but one of the most common methods is the phage display method, which comprises fusion of a protein library to the coat proteins of filamentous phage (e.g., Huse et al.,'89; Clackson et al.,'91; Marks et al., '92). Fusions are made most commonly to a minor coat protein, called the gene III protein (pIII), which is present in three to five copies at the tip of the phage. The fused library is then displayed on the surface of the viral particle. The page display library can then be screened against an immobilized target protein. However, one major drawback of this method is that target proteins that bind the library with very high affinity are not always identified because the conditions required to elute the bound phase usually denature the phage particle such that it becomes impossible to identify the protein of interest. Another draw back of phage display libraries is the requirement that the target protein be immobilized on a solid surface, which can lead to difficulties in determining the actual affinity of a target protein for the phage display protein. Furthermore, some proteins of interest require post-translational modifications, such as glycosylation, methylation, or disulfide binding, that cannot be achieved when expressed in a phage particle.

An alternative method for screening protein libraries is to display the library on the surface of bacterial cells. This method solves many of the drawbacks associated with phage display, but has its own problems. One problem with bacterial display is that the bacterial capsule can cause steric hindrance to proteins displayed on the bacterial surface. Also, bacteria do not contain the machinery to properly fold eukaryotic proteins, so the protein of interest may not always be expressed within the bacterium. Similar to the problem in phage, bacteria cannot provide post-translational modifications, like disulfide binding, to a eukaryotic protein.

Wittrup et al. (WO9936569) have developed a method for a yeast cell display library. This is a two component system, wherein the first component involves expressing one subunit of the yeast mating adhesion protein, agglutinin, which is anchored to the yeast cell wall. The second component involves expressing a protein library fused to a second subunit of the agglutinin protein which forms high affinity disulfide bonds to the first agglutinin subunit. The protein library fused to the agglutinin is thus displayed on the surface of the cell. The library can then be screened. This method allows for the proper folding and post-translational modification of eukaryotic proteins. The patent application WO2007046825 discloses methods for detecting soluble antigens using sensor cells expressing a Fc gamma Receptor I and a molecule called aequorin, which emits a photon in response to increased cell calcium concentrations. Crosslinking of the antibody-bound receptors by a specific antigen on the sensor cell results in an increase in intracellular calcium concentration and the aequorin molecules thereby emit photons in response to the increase in calcium concentration. Regis Cebe et al, disclose an in vitro selection procedure for non-infectious phages displaying a ligand able to interact with a specific biotinylated target molecule. The streptavidin domain of an engineered adapter serves as link between a biotinylated target molecule and the noninfectious phage lacking a functional gene III coat protein but displaying an interacting ligand instead (Regis et al., Size of the ligand complex between the N-terminal domain of the gene III coat protein and the non-infectious phage strongly influences the usefulness of in vitro selective infective phage technology, Biochemical Journal, volume: 352, pages: 841-849)

Rakestraw et al. (PCT/US2008/003978) have developed a three component system for displaying a protein library on the surface of yeast cells. The first component involves expressing a protein library fused to a biotin-binding peptide, the second component involves modifying the yeast cell wall to express biotin, and the third component involves binding avidin to the biotin expressed on the cell surface. The fused protein library is then biotinylated and secreted from the yeast cell and binds to the avidin on the yeast cell surface, thus displaying the protein library on the surface of the yeast cell. One potential drawback of this system is that avidin non-specifically binds all biotin. Another potential drawback is that avidin contains four binding sites, which may cause steric hindrance thus preventing the biotinylated protein library from binding to the cell surface bound avidin. Similarly, the avidin molecule may bind the biotinylated protein library before binding the biotinylated yeast cell wall, thereby hindering the binding of the avidin to the yeast cell wall. Additionally, this method contains the added complication of having to biotinylate the protein library within the yeast cell. This necessary extra step requires further modification to the yeast cell. It is well established that the more modifications are made to a biological system, the less likely it is that the system will behave as designed. In addition, since avidin/streptavidin is multivalent, care must be taken to not cross-link the biotinylated cells. Finally, biotin/streptavidin and biotin/avidin are used in a number of commercially available labeling kits. Such kits would be difficult to use in such a biotin/avidin display system.

The prior art lacks a simple, efficient system capable of specifically binding a secreted protein library using an adapter molecule that binds to the protein library and to the surface of a eukaryotic cell through different binding moieties.

### SUMMARY OF THE INVENTION

The present invention meets this need by providing methods and compositions as disclosed through out the specification. One aspect includes a library of host cells with a cell surface molecule attached to the surface of the cell, an adapter molecule comprising a first binding site and a second binding site, and a display molecule comprising a modified polypeptide selected from the group consisting of a scaffold protein, a signal transduction protein, an antibody, an immunoglobulin, an immunoadhesin, a receptor, a ligand, an oncoprotein, a transcription factor, an enzyme, and a fibronectin polypeptide, where the first binding site binds specifically to the cell surface molecule and cannot bind to the display molecule, the second binding site binds specifically to the display molecule and cannot bind to the cell surface molecule, and the adapter molecule is not a component of the modified polypeptide wherein each host cell includes a different modified polypeptide. In certain embodiments, the host cell has a plurality of display molecules. In other embodiments which may be combined with the preceding embodiments, the host cell surface molecule may be covalently linked to the first binding site. In other embodiments which may be combined with any of the preceding embodiments, the host cell surface molecule may include a first agglutinin which may be Aga1p and wherein the first binding site comprises a second agglutinin that is Aga2p. In other embodiments which may be combined with any of the preceding embodiments, the host cell surface molecule may be attached to the cell membrane via a GPI anchor. In other embodiments which may be combined with any of the preceding embodiments, the second binding site may be covalently linked to the display molecule. In other embodiments which may be combined with any of the preceding embodiments, the second binding site may be covalently linked to the display molecule through disulfide bonds. In other embodiments which may be combined with any of the preceding embodiments, the second binding site includes a PDZ domain of InaD which may have the amino acid sequence of SEQ ID NO: 8. In other embodiments which may be combined with any of the preceding embodiments, the display molecule includes a NorpA ligand which may be at the C-terminus which may have the amino acid sequence of SEQ ID NO: 9. In other embodiments which may be combined with any of the preceding embodiments except where the second binding site includes a PDZ domain or where the display molecule comprises a NorpA ligand, the display molecule includes a PDZ domain which may be the PDZ domain of InaD which may have the amino acid sequence of SEQ ID NO: 8. In other embodiments which may be combined with any of the preceding embodiments except where the second binding site includes a PDZ domain or where the display molecule comprises a NorpA ligand, the second binding site includes a NorpA ligand which may be at the C-terminus which may have the amino acid sequence of SEQ ID NO: 9. In other embodiments which may be combined with any of the preceding embodiments, wherein the modified polypeptide may be a scaffold protein, a signal transduction protein, an antibody, an immunoglobulin, an immunoadhesin, a receptor, a ligand, an oncoprotein, a transcription factor, or an enzyme. In other embodiments which may be combined with any of the preceding embodiments, display molecule may be a fibronectin polypeptide which may include an F10 polypeptide. In other embodiments which may be combined with any of the preceding embodiments, the display molecule includes a secretion signal peptide may be an MFalpha-secretion signal sequence, a glucoamylase, an Aga2 secretion signal sequence, an Flo1p secretion signal sequence, an invertase secretion signal sequence, or an acid phosphatase secretion signal sequence. In other embodiments which may be combined with any of the preceding embodiments, the secretion signal peptide may be an MFalpha/HSA hybrid leader peptide. In other embodiments which may be combined with any of the preceding embodiments, expression of the display molecule is under the control of a first inducible promoter which may be an AOX 1 promoter, a Cup 1 promoter, or a Gal promotor. In other embodiments which may be combined with any of the preceding embodiments, the expression of the adapter molecule is under the control of a second inducible promoter which may be an AOX 1 promoter, a Cup 1 promoter, or a Gal promotor. In other embodiments which may be combined with any of the preceding embodiments, the host cell may be a yeast cell which may be *Pichia pastoris* or *Saccharomyces cerevisiae.*

Yet another aspect includes methods for displaying a modified polypeptide which includes (a) providing a host cell comprising a cell surface molecule attached to the surface of the cell which is expressed from an expression vector in the host cell and a first nucleic acid encoding a display polypeptide comprising a modified polypeptide selected from the group consisting of a scaffold protein, a signal transduction protein, an antibody, an immunoglobulin, an immunoadhesin, a receptor, a ligand, an oncoprotein, a transcription factor, an enzyme, and a fibronectin polypeptide,, (b) contacting the host cell with an adapter molecule comprising a first binding site and a second binding site under conditions wherein the first binding site binds to the cell surface molecule, and then (c) incubating the host cell under conditions wherein the host cell exports the display polypeptide outside the host cell under conditions wherein the second binding site binds to the display polypeptide, where the first binding site binds specifically to the cell surface molecule and cannot bind to the display molecule, the second binding site binds specifically to the display polypeptide and cannot bind to the cell surface molecule, and the adapter molecule is not a component of the modified polypeptide. In other embodiments which may be combined with the preceding embodiments, the host cell surface molecule may be covalently linked to the first binding site. In other embodiments which may be combined with the preceding embodiments, the host cell surface molecule may be covalently linked to the first binding site through a disulfide bond. In other embodiments which may be combined with any of the preceding embodiments, the host cell surface molecule may include a first agglutinin which may be Aga1p. In other embodiments which may be combined with any of the preceding embodiments, the host cell surface molecule may be attached to the cell membrane via a GPI anchor. In other embodiments which may be combined with any of the preceding embodiments, wherein the first binding site comprises a second agglutinin which may be Aga2p. In other embodiments which may be combined with any of the preceding embodiments, the second binding site may be covalently linked to the display molecule. In other embodiments which may be combined with any of the preceding embodiments, the second binding site may be covalently linked to the display molecule through disulfide bonds. In other embodiments which may be combined with any of the preceding embodiments, the second binding site includes the PDZ domain of InaD which may have the amino acid sequence of SEQ ID NO: 8. In other embodiments which may be combined with any of the preceding embodiments, the display molecule includes a NorpA ligand which may be at the C-terminus which may have the amino acid sequence of SEQ ID NO: 9. In other embodiments which may be combined with any of the preceding embodiments except where the second binding site includes a PDZ domain or where the display molecule comprises a NorpA ligand, the display molecule includes a PDZ domain which may be the PDZ domain of InaD which may have the amino acid sequence of SEQ ID NO: 8. In other embodiments which may be combined with any of the preceding embodiments except where the second binding site includes a PDZ domain or where the display molecule comprises a NorpA ligand, the second binding site includes a NorpA ligand which may be at the C-terminus which may have the amino acid sequence of SEQ ID NO: 9. In other embodiments which may be combined with any of the preceding embodiments, wherein the modified polypeptide may be a scaffold protein, a signal transduction protein, an antibody, an immunoglobulin, an immunoadhesin, a receptor, a ligand, an oncoprotein, a transcription factor, or an enzyme. In other embodiments which may be combined with any of the preceding embodiments, display molecule may be a fibronectin polypeptide which may include an F10 polypeptide. In other embodiments which may be combined with any of the preceding embodiments, the display molecule includes a secretion signal peptide may be an MFalpha secretion signal sequence, a glucoamylase, an Aga2 secretion signal sequence, an Flo1p secretion signal sequence, an invertase secretion signal sequence, or an acid phosphatase secretion signal sequence. In other embodiments which may be combined with any of the preceding embodiments, the secretion signal peptide may be an MFalpha/HSA hybrid leader peptide. In other embodiments which may be combined with any of the preceding embodiments, expression of the display molecule is under the control of a first inducible promoter which may be an AOX 1 promoter,a Cup 1 promoter, or a Gal promotor. In other embodiments which may be combined with any of the preceding embodiments, the expression of the adapter molecule is under the control of a second inducible promoter which may be an AOX 1 promoter, a Cup 1 promoter, or a Gal promotor. In other embodiments which may be combined with any of the preceding embodiments, the host cell may be a yeast cell which may be *Pichia pastoris* or *Saccharomyces cerevisiae.*

Still another aspect includes methods for generating a host cell display library which includes introducing into a plurality of host cells a display library of first nucleic acids each encoding a display polypeptide comprising a modified polypeptide selected from the group consisting of a scaffold protein, a signal transduction protein, an antibody, an immunoglobulin, an immunoadhesin, a receptor, a ligand, an oncoprotein, a transcription factor, an enzyme, and a fibronectin polypeptide, wherein at least two of the introduced first nucleic acids encode different modified polypeptides, wherein each host cell comprises a second nucleic acid which encodes a cell surface polypeptide and a third nucleic acid which encodes an adapter molecule comprising a first binding site and a second binding site, where the first binding site binds to the cell surface molecule but not the display polypeptide, the second binding site binds to the display polypeptide but not the cell surface molecule, and the adapter molecule is not a component of the modified polypeptide. This aspect may be combined with any of the embodiments of the preceding aspects.

The foregoing are non-limiting examples of the present invention. Additional aspects and embodiments may be found throughout the specification.

### BRIEF DESCRIPTION OF THE DRAWINGS

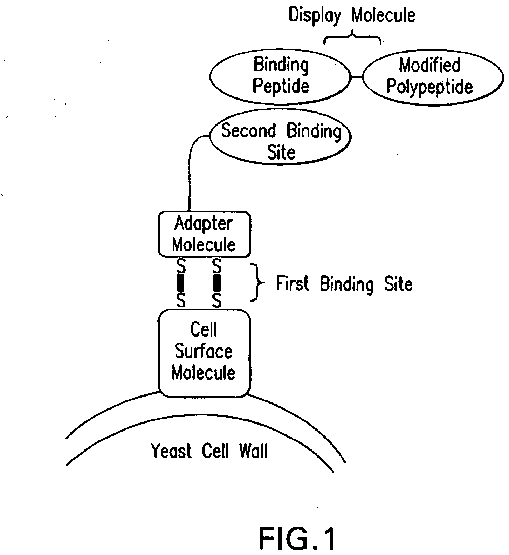

Figure 1 shows a schematic of the three component system including the cell surface molecule, the adapter molecule including the first and second binding sites and the display molecule with the binding partner for the second binding site (a binding polypeptide in this embodiment) and a modified polypeptide as the molecule being displayed. In this embodiment, the host cell is a yeast cell.

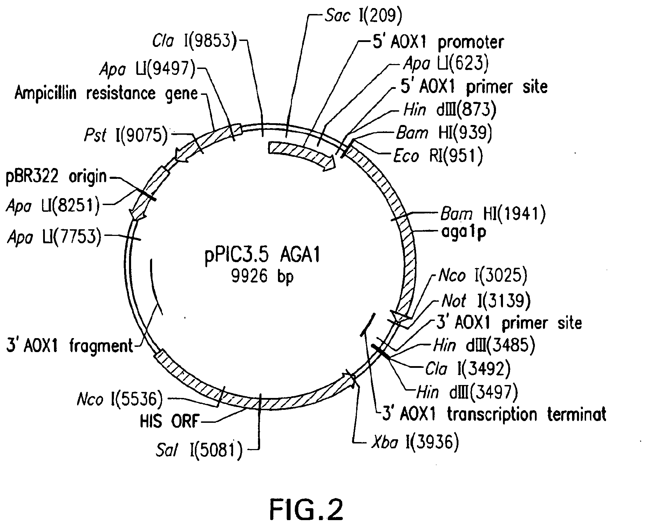

Figure 2 shows the pPIC3.5 AGA1 vector for yeast expression of the Aga1p as the cell surface molecule.

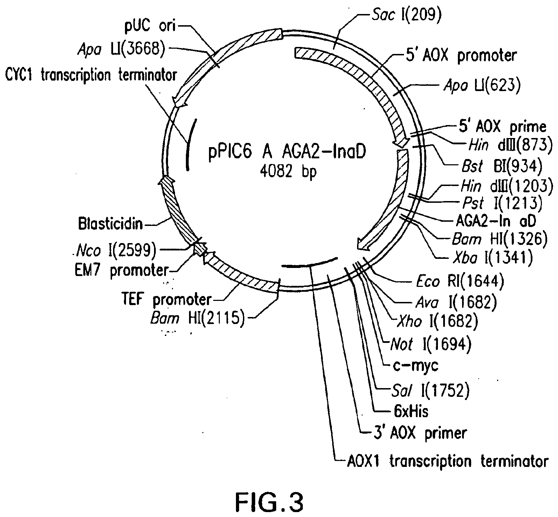

Figure 3 shows the pPIC6 A AGA2-InaD vector for yeast expression of the Aga2p-InaD fusion polypeptide as the adapter molecule.

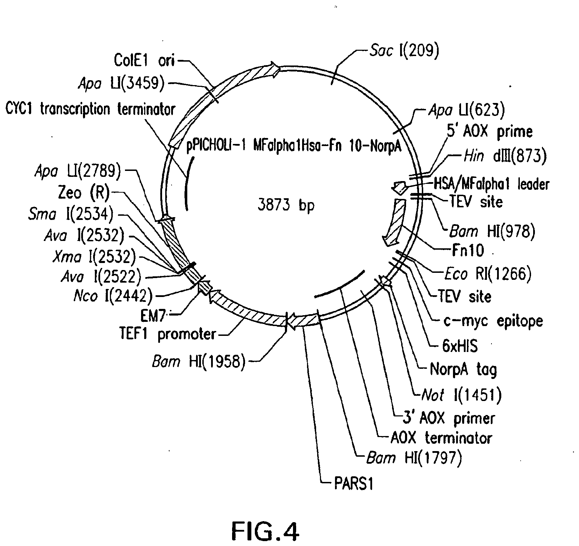

Figure 4 shows the pPICHOLI-1 MFalpha1Hsa-Fn10-NorpA vector for yeast expression of the MFalpha1Hsa-Fn 10-NorpA fusion polypeptide as the display molecule where the NorpA is the binding partner of the second binding site of the adapter molecule (i.e., InaD) and the fibronectin F10 domain is modified polypeptide.

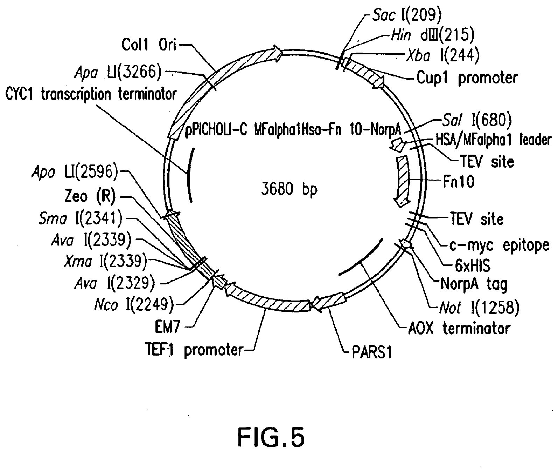

Figure 5 shows the pPICHOLI-C MFalpha1Hsa-Fn10-NorpA vector for yeast expression of the MFalpha1Hsa-Fn10-NorpA fusion polypeptide as the display molecule where the NorpA is the binding partner of the second binding site of the adapter molecule (i.e., InaD) and the fibronectin F10 domain is modified polypeptide.

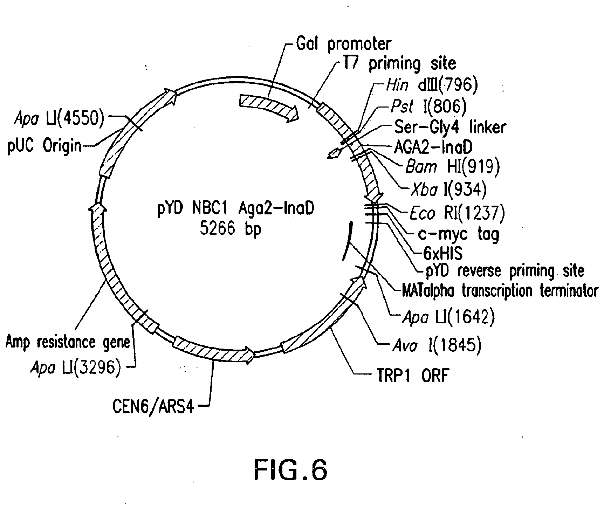

Figure 6 shows the pYD NBC1 Aga2-InaD vector for yeast expression of the Aga2p-InaD fusion polypeptide as the adapter molecule.

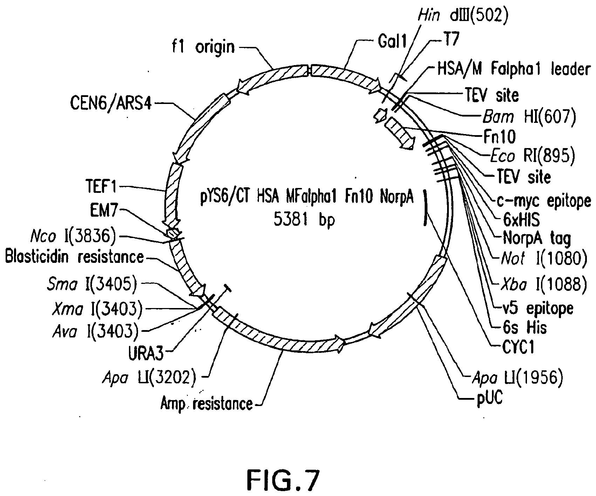

Figure 7 shows the pYS HSA MFalpha1 Fn10 NorpA vector for yeast expression of the MFalpha1HSA-Fn10-NorpA fusion polypeptide as the display molecule where the NorpA is the binding partner of the second binding site of the adapter molecule (i.e., InaD) and the fibronectin F10 domain is modified polypeptide.

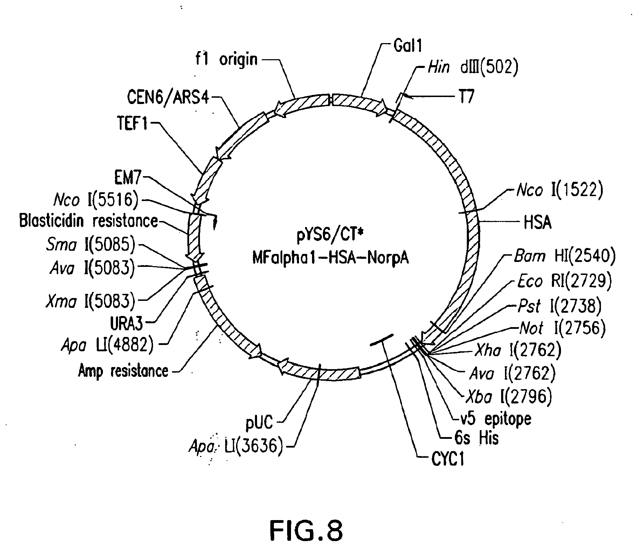

Figure 8 shows the pYS MFalpha1 HSA NorpA vector for yeast expression of the MFalpha1HSA--NorpA fusion polypeptide as the display molecule where the NorpA is the binding partner of the second binding site of the adapter molecule (i.e., InaD) and the HSA is the displayed polypeptide.

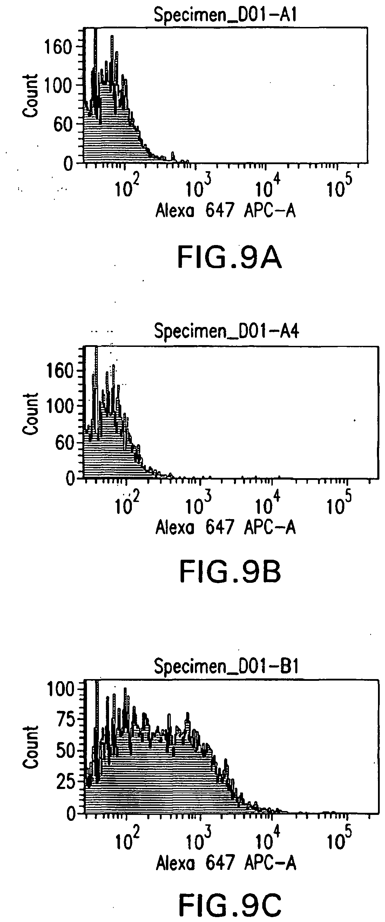

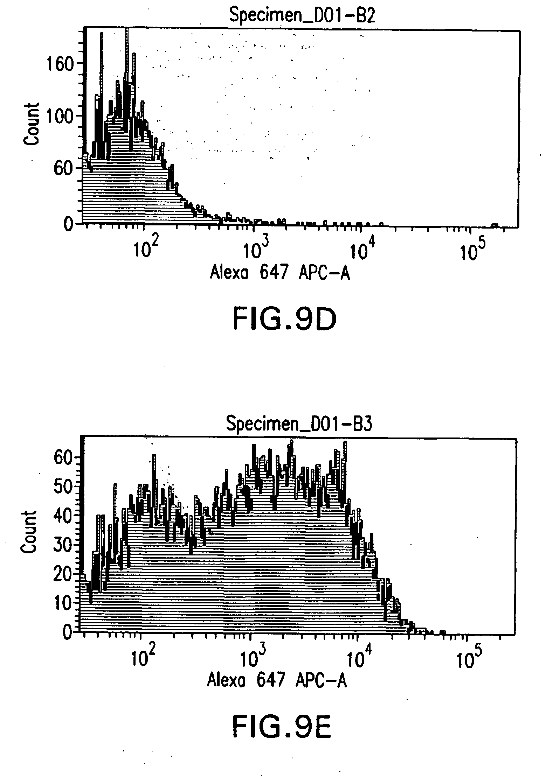

Figure 9A-E are FACS analysis graphs showing protein surface expression of yeast cells expressing either fibronectin (pYS6/CT HSA MFalpha1 Fn10 NorpA) or HSA (pYS6/CT MFalpha1-HSA-NorpA) in which the yeast cells are stained with anti-myc antibody and APC labeled secondary anti-mouse antibody. Figure 9A is the a control with unstained yeast cells: Figure 9B are uninduced yeast cells expressing fibronectin; Figure 9C are induced yeast cells expressing fibronectin showing a shift in the curve; Figure 9D are uninduced yeast cells expressing HSA; and Figure 9E are induced yeast cells expressing HSA showing a shift in the curve.

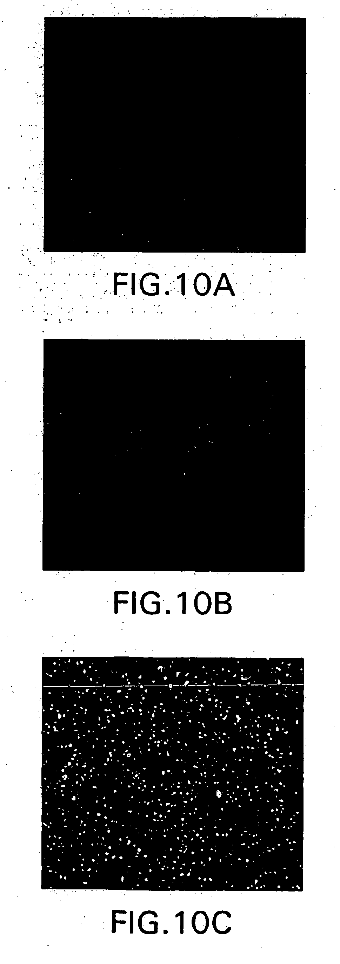

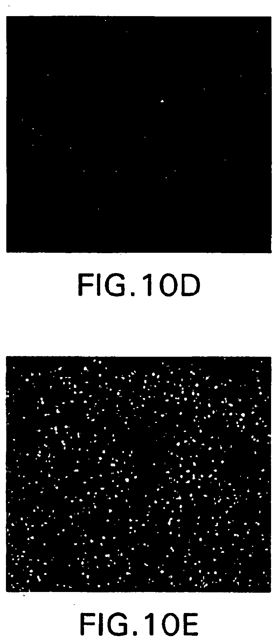

Figure 10A-E are images of FMAT analysis of yeast cells expressing either fibronectin (pYS6/CT HSA MFalpha1 Fn10 NorpA) or HSA (pYS6/CT HSA-NorpA) in which the yeast cells are stained with anti-myc mouse monoclonal antibody and APC labeled secondary anti-mouse antibody and subjected to FMAT confocal fluorescence. Figure 10A is the a control with unstained yeast cells: Figure 10B are uninduced yeast cells expressing fibronectin; Figure 10C are induced yeast cells expressing fibronectin appearing as white spots; Figure 10D are uninduced yeast cells expressing HSA; and Figure 10E are induced yeast cells expressing HSA appearing as white spots.

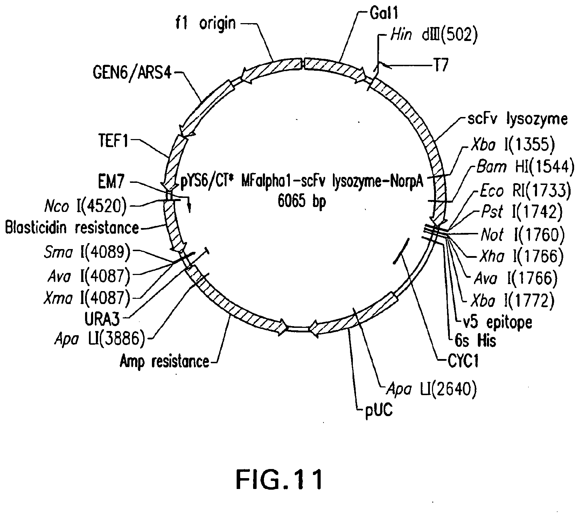

Figure 11 shows the pYS MFalpha1 scFv lysozyme NorpA vector for yeast expression of the MFalpha1 scFv lysozyme NorpA fusion polypeptide as the display molecule where the NorpA is the binding partner of the second binding site of the adapter molecule (i.e., InaD) and the lysozyme is modified polypeptide.

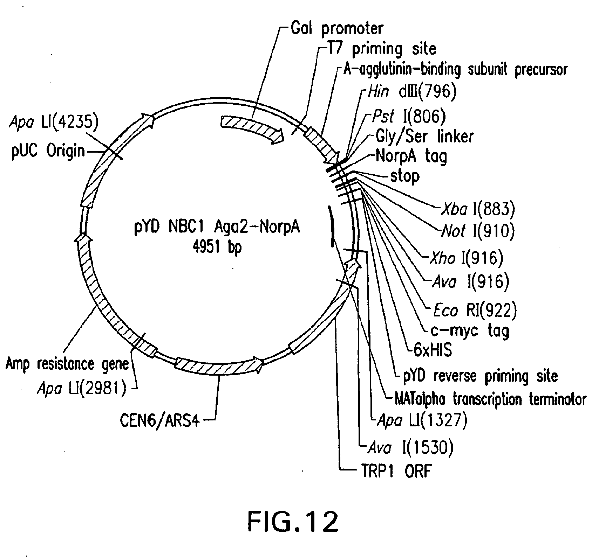

Figure 12 shows a reversed system in which the pYD NBC 1 Aga2-NorpA vector is used for yeast expression of the NBC1 Aga2-NorpA.

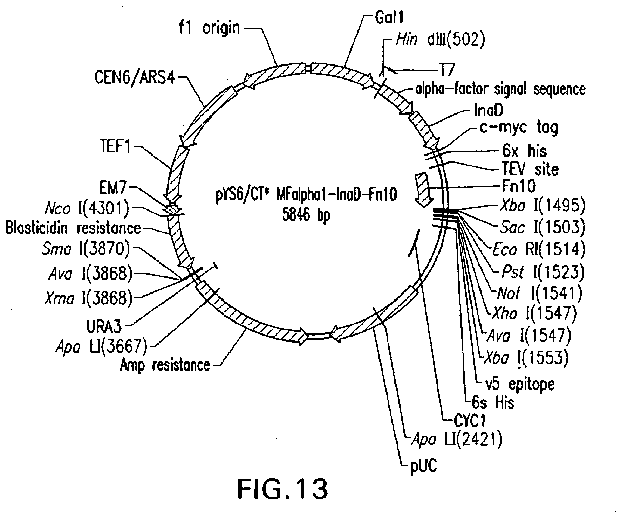

Figure 13 shows a reversed system in which the pYS6/CT*MFalpha1-InaD-Fn10 vector is used for yeast expression of the MFalpha1-InaD-Fn10.

### DETAILED DESCRIPTION OF THE INVENTION

### Definitions

As used herein the term "host cell" refers to a eukaryotic cell that has been modified to express a cell surface molecule, an adapter molecule, and a display molecule. Furthermore, it should be understood that the host cell secretes or excretes the display molecule prior to binding the display molecule to the adapter molecule on the surface of the host cell.

As used herein the term "cell surface molecule" refers to a peptide, polypeptide, binding domain, ligand, lipid, or carbohydrate that is directed to the extracellular surface of the host cell. The cell surface molecule may be anchored to the cell surface by covalent binding or non-covalent binding. The cell surface molecule may include a phospholipid, carbohydrate, or protein through which it attaches to the surface of the host cell. The cell surface molecule may be a polypeptide that binds to, or is conjugated to, a phospholipid, carbohydrate, or a polypeptide on the surface of the cell. For example, the polypeptide may use a phosphatidyl-inositol-glycan (GPI) anchor to attach to the surface of the host cell, such as a-agglutinins, α-agglutinins, and flocculins. The cell surface molecule may also be a transmembrane protein with a binding domain located on the surface of the host cell that can bind to the first binding site of the adapter molecule.

As used herein the term "adapter molecule" refers to a peptide, polypeptide, binding domain, ligand, lipid, or carbohydrate or combination of the foregoing that has two distinct binding sites. The adapter molecule has a binding site that specifically binds the cell surface molecule and a second distinct binding site that specifically binds the display molecule. Without limiting the invention, the two binding sites of the polypeptide may be polypeptide domains each with its own binding affinity to a different molecule that are fused together. For example, the polypeptide may be an a-agglutinin subunit, such as Aga2p, fused to a PDZ domain, or it may be a flocculin, such as Flo1, fused to a PDZ domain.

As used herein the term "first binding site" refers to a region of the adapter molecule that specifically recognizes and binds at least a portion of the cell surface molecule. For example, the first binding site may comprise a peptide, polypeptide, binding domain, ligand, lipid, or carbohydrate or combination thereof that specifically binds to a cell surface molecule which could include, without limitation, a peptide, binding domain, ligand, protein, lipoprotein, lipid, or carbohydrate. More specifically, but without limiting the invention, the first binding site may refer to the Aga2p subunit of a-agglutinin that specifically binds to the Aga1p subunit of a-agglutinin through disulfide bonds. In general, any two molecular binding partners may be used for the first binding site and the corresponding portion of the cell surface molecule. Examples include Ni²⁺ ions and polyhistidine tags, sugar residues and Concanavalin A, p-aminophenyl-β-D-thiogalactoside and β-galactosidase (Germino et al., Proc. Natl. Acad. Sci. USA 80:6848 (1983), glutathione and glutathione-S-transferase (Smith, D. B. and Johnson, K. S. Gene 67:31 (1988)); staphylococcal protein A and IgG (Uhlen, M. et al. Gene 23:369 (1983)), calmodulin nickel binding proteins (CNBP) and calmodulin agarose (Stofko-Hahn, R. E. et al. FEBS Lett. 302(3):274-278); streptavidin or avidin and biotin (Takashige, S. and Dale, G. L., Proc. Natl. Acad. Sci. USA. 85:1647-1651 (1988)); amylase and maltose-binding protein domain from the malE gene of E. coli (Bach, H. et al., J. Mol. Biol. 312:79-93 (2001)), any epitope and its corresponding antibody (See, Kolodziej, P. A. and Young, R. A., Methods Enzymol. 194:508-519 (1991), e.g., the FLAG(TM) octapeptide or antidigoxygenin antibody and digoxygenin).

As used herein the term "second binding site" refers to a region of the adapter molecule that specifically recognizes and binds the display molecule. For example, the second binding site may comprise a peptide, polypeptide, binding domain, ligand, lipid, or carbohydrate that specifically binds to a peptide, ligand, protein, lipoprotein, lipid, or carbohydrate comprising the display molecule. More specifically, but without limitations, the second binding site may refer to a PMZ domain that specifically binds to a NorpA ligand. Any of the binding pairs suitable for the first binding site may also be used for the second binding site so long as they do not recognize the same partners.

As used herein the term "display molecule" refers to a molecule that can be localized to the surface of the host cell via binding of the adapter molecule on the surface of the host cell. The display molecule will typically comprise the molecule (or library of molecules) to be displayed and a binding partner that is specifically bound by the second binding site of the adapter molecule. In certain instances the molecule to be displayed and the binding partner may be one in the same. By way of example, the display molecule may comprise a peptide, polypeptide, binding domain, ligand, lipid, or carbohydrate or combination thereof. It should be understood that the display molecule is expressed or otherwise generated within the host cell and is secreted or excreted out of the cell so as to be displayed on the surface of said cell. The display molecule may comprise a library of varied molecules that can be screened for binding to a target or for improved or altered activity. In certain embodiments, the library may comprise modified polypeptides. The display molecule may also comprise a tag or peptide that can be labeled so as to detect binding of the display molecule to the cell surface, or sort host cells displaying said molecule.

As used herein the term "modified polypeptide" refers to any polypeptide of interest that is fused to a peptide, polypeptide, binding domain, ligand, lipid, or carbohydrate that specifically binds to a peptide, ligand, protein, lipoprotein, lipid, or carbohydrate comprising the second binding site of the adapter molecule, and is displayed on the surface of the host cell (and is therefore a component of the display molecule). The modified polypeptide is selected from the group consisting of a scaffold protein, a signal transduction protein, an antibody, an immunoglobulin, an immunoadhesin, a receptor, a ligand, an oncoprotein, a transcription factor, an enzyme, and a fibronectin polypeptide.

As used herein, the term "plurality of display molecules" refers to at least two copies of the display molecule displayed on the surface of host cells. In certain instances, each unique display molecule is displayed by a different host cell.

As used herein the term "component of the modified polypeptide" refers to any naturally occurring binding partners of any fragment of the modified peptide. Non-limiting examples include an immunoglobulin light chain binding to an immunoglobulin heavy, a biotin molecule binding avidin, two subunits of α-hemoglobin dimerizing, a myosin heavy chain binding to a myosin light chain, two monomers of glycophorin A dimerizing, or two monomers of any naturally occurring dimer protein binding to one another.

As used herein the term "library of host cells" refers to a plurality of host cells, wherein each host cell comprises a non-identical modified polypeptide that is displayed on the surface of the cell.

As used herein the term "non-identical modified polypeptide" refers to the amino acid sequence of at least two modified polypeptides, wherein each amino acid sequence comprises amino acid substitutions, insertions, or deletions which differentiate one modified polypeptide displayed on the surface of a host cell from another modified polypeptide displayed on the surface of a second host cell.

As used herein the term "Fn 10" refers to the tenth type III domain of human fibronectin.

### Display Molecules

The display molecules may be used to display any molecule that may be expressed or otherwise generated in a host cell. Non limiting examples of such molecules follow.

### Antibody scaffold

The display molecules may be immunoglobulins. Methods of generating libraries of immunoglobulins were initially developed to display the immunoglobulins via phage, but additional methods of display have since been developed. All of the methods of generating libraries for these alternative methods of display may be adapted to allow display using the methods disclosed herein. Exemplary method of generating libraries of antibodies may be found in: U.S. Patent Nos. 5,223,409; 5,403,484; and 5,571,698 to Ladner et al.; U.S. Patent Nos. 5,427,908 and 5,580,717 to Dower et al.; U.S. Patent Nos. 5,969,108 and 6,172,197 to McCafferty et al.; and U.S. Patent Nos. 5,885,793; 6,521,404; 6,544,731; 6,555,313; 6,582,915 and 6,593,081 to Griffiths et al.

### Non-antibody scaffold

Known non-immunoglobulin frameworks or scaffolds which may be displayed using the methods disclosed herein include, but are not limited to, fibronectins (Compound Therapeutics, Inc., Waltham, MA), ankyrin (Molecular Partners AG, Zurich, Switzerland), domain antibodies (Domantis, Ltd (Cambridge, MA) and Ablynx nv (Zwijnaarde, Belgium)), lipocalin (Anticalin) (Pieris Proteolab AG, Freising, Germany), small modular immuno-pharmaceuticals (Trubion Pharmaceuticals Inc., Seattle, WA), maxybodies (Avidia, Inc. (Mountain View, CA)), Protein A (Affibody AG, Sweden) and affilin (gamma-crystallin or ubiquitin) (Scil Proteins GmbH, Halle, Germany), protein epitope mimetics (Polyphor Ltd, Allschwil, Switzerland).

### (i) Fibronectins

The adnectin scaffolds are based on fibronectin type III domain (e.g., the tenth module of the fibronectin type III (10 Fn3 domain). The fibronectin type III domain has 7 or 8 beta strands which are distributed between two beta sheets, which themselves pack against each other to form the core of the protein, and further containing loops (analogous to CDRs) which connect the beta strands to each other and are solvent exposed. There are at least three such loops at each edge of the beta sheet sandwich, where the edge is the boundary of the protein perpendicular to the direction of the beta strands. (US 6,673,901).

These fibronectin-based scaffolds are not an immunoglobulin, although the overall fold is closely related to that of the smallest functional antibody fragment, the variable region of the heavy chain, which comprises the entire antigen recognition unit in camel and llama IgG. Because of this structure, the non-immunoglobulin fibronectin molecule mimics antigen binding properties that are similar in nature and affinity to those of antibodies. These scaffolds can be used in a loop randomization and shuffling strategy *in vitro* that is similar to the process of affinity maturation of antibodies *in vivo.* These fibronectin-based molecules can be used as scaffolds where the loop regions of the molecule can be replaced with CDRs of the disclosure using standard cloning techniques.

### (ii) Ankyrin - Molecular Partners

The technology is based on using proteins with ankyrin derived repeat modules as scaffolds for bearing variable regions which can be used for binding to different targets. The ankyrin repeat module is a 33 amino acid polypeptide consisting of two anti-parallel α-helices and a β-turn. Binding of the variable regions is mostly optimized by using ribosome display.

### (iii) Maxybodies/Avimers - Avidia

Avimers are derived from natural A-domain containing protein such as LRP-1. These domains are used by nature for protein-protein interactions and in human over 250 proteins are structurally based on A-domains. Avimers consist of a number of different "A-domain" monomers (2-10) linked via amino acid linkers. Avimers can be created that can bind to the target antigen using the methodology described in, for example, 20040175756; 20050053973; 20050048512; and 20060008844.

### (vi) Protein A - Affibody

Affibody® affinity ligands are small, simple proteins composed of a three-helix bundle based on the scaffold of one of the IgG-binding domains of Protein A. Protein A is a surface protein from the bacterium Staphylococcus aureus. This scaffold domain consists of 58 amino acids, 13 of which are randomized to generate Affibody® libraries with a large number of ligand variants (See e.g., US 5,831,012). Affibody® molecules mimic antibodies, they have a molecular weight of 6 kDa, compared to the molecular weight of antibodies, which is 150 kDa. In spite of its small size, the binding site of Affibody® molecules is similar to that of an antibody.

### (v) Anticalins - Pieris

Anticalins® are products developed by the company Pieris ProteoLab AG. They are derived from lipocalins, a widespread group of small and robust proteins that are usually involved in the physiological transport or storage of chemically sensitive or insoluble compounds. Several natural lipocalins occur in human tissues or body liquids.

The protein architecture is reminiscent of immunoglobulins, with hypervariable loops on top of a rigid framework. However, in contrast with antibodies or their recombinant fragments, lipocalins are composed of a single polypeptide chain with 160 to 180 amino acid residues, being just marginally bigger than a single immunoglobulin domain.

The set of four loops, which makes up the binding pocket, shows pronounced structural plasticity and tolerates a variety of side chains. The binding site can thus be reshaped in a proprietary process in order to recognize prescribed target molecules of different shape with high affinity and specificity.

One protein of lipocalin family, the bilin-binding protein (BBP) of Pieris Brassicae has been used to develop anticalins by mutagenizing the set of four loops. One example of a patent application describing "anticalins" is PCT WO 199916873.

### (vi) Affilin - Scil Proteins

Affilin™ molecules are small non-immunoglobulin proteins which are designed for specific affinities towards proteins and small molecules. New Affilin ™ molecules can be very quickly selected from two libraries, each of which is based on a different human derived scaffold protein.

Affilin™ molecules do not show any structural homology to immunoglobulin proteins. Scil Proteins employs two Affilin™ scaffolds, one of which is gamma crystalline, a human structural eye lens protein and the other is "ubiquitin" superfamily proteins. Both human scaffolds are very small, show high temperature stability and are almost resistant to pH changes and denaturing agents. This high stability is mainly due to the expanded beta sheet structure of the proteins. Examples of gamma crystalline derived proteins are described in WO200104144 and examples of "ubiquitin-like" proteins are described in WO2004106368.

### (vii) Protein Epitope Mimetics (PEM)

PEM are medium-sized, cyclic, peptide-like molecules (MW 1-2kDa) mimicking beta-hairpin secondary structures of proteins, the major secondary structure involved in protein-protein interactions.

### Non-scaffold

In addition to scaffolds which are useful for de novo generation of molecule with specific affinity, the methods disclosed herein may be used to display any other biological molecule that can be expressed or otherwise generated in the host cell. Libraries of such biological molecules, particularly polypeptides which can be encoded by polynucleotides for easy expression by the host cell, may be screened for improved characteristics of interest such as improved binding between receptor and ligand where the receptor or the ligand are part of the display molecule or improved enzymatic activity where the enzyme is part of the display molecule.

### Expression Systems

Expression vectors may be used to express one or more of the cell surface molecules, the adapter molecule and the display molecule, in the host cell. Expression vectors for eukaryotic host cells typically include (i) eukaryotic DNA elements that control initiation of transcription, such as a promoter, (ii) eukaryotic DNA elements that control the processing of transcripts, such as a transcription termination/polyadenylation signal sequence, and (iii) optionally, eukaryotic DNA elements that control replication in the eukaryotic host cell if the vector is to be independently replicated (e.g., non-integrating vectors). To ease construction of such expression vectors, the vectors may optionally include (iv) prokaryotic DNA elements coding for a bacterial replication origin and an antibiotic resistance marker to provide for the growth and selection of the expression vector when manipulating the vector in the bacterial host cell. Appropriate eukaryotic expression vectors for use with fungal, yeast, and mammalian cellular hosts are known in the art, and are described in, for example, Powels et al. (Cloning Vectors: A Laboratory Manual, Elsevier, N.Y., 1985).

Yeast host cells are of particular interest and include Saccharomyces cerevisiae, Pichia pastoris, and Pichia methanolica. These vectors include YIp-based vectors, such as YIp5, YRp vectors, such as YRp 17, YEp vectors such as YEp13 and YCp vectors, such as YCp 19. A number of vectors exist for the expression of recombinant proteins in yeast. Other example of the YEp vectors include YEp24, YEp51, and YEp52,which are cloning and expression vehicles useful in the introduction of genetic constructs into S. cerevisiae (see, e.g., Broach et al. (1983) in Experimental Manipulation of Gene Expression, ed. M. Inouye Academic Press, p. 83). These vectors are also shuttle vectors in that they can replicate in E. coli due the presence of the pBR322 ori, and in S. cerevisiae due to the replication determinant of the yeast 2 micron plasmid.

Suitable promoters for function in yeast include the promoters for metallothionein, 3-phosphoglycerate kinase (Hitzeman et al., J. Biol. Chem. 255, 2073 (1980) or other glycolytic enzymes (Hess et al., J. Adv. Enzyme Req. 7, 149 (1968); and Holland et al. Biochemistry 17, 4900 (1978)), such as enolase, glyceraldehyde-3-phosphate dehydrogenase, hexokinase, pyruvate decarboxylase, phospho-fructokinase, glucose-6-phosphate isomerase, 3-phosphoglycerate mutase, pyruvate kinase, triosephosphate isomerase, phospho-glucose isomerase, and glucokinase. Suitable vectors and promoters for use in yeast expression are further described in R. Hitzeman et al., EP073,657. Other suitable promoters for expression in yeast include the promoters from GAL 1 (galactose), PGK (phosphoglycerate kinase), ADH (alcohol dehydrogenase), AOX1 (alcohol oxidase), HIS4 (histidinol dehydrogenase), and the like. Many yeast cloning vectors readily available and can be modified following the above discussion. Still other promoters, which have the additional advantage of transcription controlled by growth conditions, are the promoter regions for alcohol dehydrogenase 2, isocytochrome C, acid phosphatase, degradative enzymes associated with nitrogen metabolism, and the afore-mentioned metallothionein and glyceraldehyde-3-phosphate dehydrogenase, as well as enzymes responsible for maltose and galactose utilization. Finally, promoters that are active in only one of the two haploid mating types may be appropriate in certain circumstances. Among these haploid-specific promoters, the pheromone promoters MFa1 and Meα1 are of particular interest.

Secretion from yeast host cells of the components including the adapter molecule (if produced in the host cell) and the display molecule may be increased by use any available secretion signal sequences of yeast proteins. One example is the leader sequence of a precursor of yeast mating pheromone, α-factor, which has also been used to direct secretion of heterologous proteins in yeast (See, e.g., Valenzuela, P., eds pp. 269-280, Butterworths, London; Brake, A. J. (1990) Meth. Enzymol. 185, 408-441). The α-factor leader sequence, in addition to the N-terminal signal peptide of 17 residues, includes a hydrophilic pro-region which contains 72 residues and bears three sites of N-linked glycosylation. The pro-region is extensively glycosylated in the ER and Golgi and is cleaved by Kex2 endopeptidase in the late Golgi compartment. The presence of the pro-region at the N-terminus is believed to allow some heterologous proteins to pass the quality control in the ER and to reach the periplasm.

Another example is the leader sequence from yeast invertase (MLLQAFLFLLAGFAAKISADAHKS) (SEQ ID NO: 1). This leader sequence has been demonstrated to be cleaved from nascent heterologous peptide upon entrance into the endoplasmic reticulum. The enzyme responsible for cleavage of the pre sequence, Kex2, resides in the trans Golgi. A further example is the signal sequence of yeast acid phosphatase which may be used to direct the secretion of the components disclosed herein.

Methods for transforming S. cerevisiae cells with exogenous DNA and producing recombinant polypeptides therefrom are disclosed by, for example, Kawasaki, U.S. Pat. No. 4,599,311, Kawasaki et al., U.S. Pat. No. 4,931,373, Brake, U.S. Pat. No. 4,870,008, Welch et al., U.S. Pat. No. 5,037,743, and Murray et al., U.S. Pat. No. 4,845,075. Transformed cells are selected by phenotype determined by the selectable marker, commonly drug resistance or the ability to grow in the absence of a particular nutrient (e.g., leucine). A preferred vector system for use in Saccharomyces cerevisiae is the POT1 vector system disclosed by Kawasaki et al. (U.S. Pat. No. 4,931,373), which allows transformed cells to be selected by growth in glucose-containing media.

Transformation systems for other yeasts, including Hansenula polymorpha, Schizosaccharomyces pombe, Kluyveromyces lactis, Kluyveromyces fragilis, Ustilago maydis, Pichia pastoris, Pichia methanolica, Pichia guillermondii and Candida maltosa are known in the art. See, for example, Gleeson et al., J. Gen. Microbiol. 132:3459 (1986), and Cregg, U.S. Pat. No. 4,882,279. Aspergillus cells may be utilized according to the methods of McKnight et al., U.S. Pat. No. 4,935,349. Methods for transforming Acremonium chrysogenum are disclosed by Sumino et al., U.S. Pat. No. 5,162,228. Methods for transforming Neurospora are disclosed by Laambowitz, U.S. Pat. No. 4,486,533.

For example, the use of Pichia methanolica as host for the production of recombinant proteins is disclosed by Raymond, U.S. Pat. No. 5,716,808, Raymond, U.S. Pat. No. 5,736,383, Raymond et al., Yeast 14:11-23 (1998), and in international publication Nos. WO 97/17450, WO 97/17451, WO 98/02536, and WO 98/02565. DNA molecules for use in transforming P. methanolica will commonly be prepared as double-stranded, circular plasmids, which are preferably linearized prior to transformation. For polypeptide production in P. methanolica, it is preferred that the promoter and terminator in the plasmid be that of a P. methanolica gene, such as a P. methanolica alcohol utilization gene (AUG 1 or AUG2). Other useful promoters include those of the dihydroxyacetone synthase (DHAS), formate dehydrogenase (FMD), and catalase (CAT) genes. To facilitate integration of the DNA into the host chromosome, it is preferred to have the entire expression segment of the plasmid flanked at both ends by host DNA sequences. For large-scale, industrial processes where it is desirable to minimize the use of methanol, it is preferred to use host cells in which both methanol utilization genes (AUG1 and AUG2) are deleted. For production of secreted proteins, host cells deficient in vacuolar protease genes (PEP4 and PRB1) are preferred. Electroporation is used to facilitate the introduction of a plasmid containing DNA encoding a polypeptide of interest into P. methanolica cells. P. methanolica cells can be transformed by electroporation using an exponentially decaying, pulsed electric field having a field strength of from 2.5 to 4.5 kV/cm, preferably about 3.75 kV/cm, and a time constant (t) of from 1 to 40 milliseconds, most preferably about 20 milliseconds.

For use of mammalian host cells, mammalian expression vectors are also well known in the art and may be used as well. Examples of suitable mammalian host cells include African green monkey kidney cells (Vero; ATCC CRL 1587), human embryonic kidney cells (293-HEK; ATCC CRL 1573), baby hamster kidney cells (BHK-21, BHK-570; ATCC CRL 8544, ATCC CRL 10314), canine kidney cells (MDCK; ATCC CCL 34), Chinese hamster ovary cells (CHO-K1; ATCC CCL61; CHO DG44 (Chasin et al., Som. Cell. Molec. Genet. 12:555, 1986)), rat pituitary cells (GH1; ATCC CCL82), HeLa S3 cells (ATCC CCL2.2), rat hepatoma cells (H4-II-E; ATCC CRL 1548) SV40-transformed monkey kidney cells (COS-1; ATCC CRL 1650) and murine embryonic cells (NIH-3T3; ATCC CRL 1658).

### EXAMPLES

The following provides non-limiting examples of the systems, compositions and methods disclosed herein. Proteins can be displayed on the surface of yeast cells by utilizing the PDZ domain of the yeast InaD protein and the C-terminal 5 amino acids of the yeast NorpA protein. This three component protein display system consists of a vector expressing the protein to be displayed with a secretion signal fused at its N-terminus, and the NorpA ligand fused at the C-terminus; a second vector expressing an adapter protein that can bind specifically to a yeast cell wall protein, and which is fused to the PDZ domain of InaD, which binds specifically to the NorpA ligand; and a third vector that express a yeast cell wall protein that binds specifically to the adapter protein. This system has been adapted for use in *Sacchromyces cerrevisiae* and *Pichia pastoris.*

### Example 1: Yeast display using InaD/NorpA interaction in Pichia pastoris

The protein display system for *P. pastoris* was developed to display a fibronectin type III domain (Fn 10), by fusing a hybrid secretion sequence (MFalpha/HSA) or a yeast leader sequence (MFalpha1) at N-terminus of Fn10 and fusing the NorpA ligand to its C-terminus. Once expressed, the Fn 10 was secreted from the cell and the NorpA ligand bound specifically to the PDZ domain of InaD through disulfide bonds. The InaD was fused to the C-terminus of the Agap2 protein. The Aga2p-InaD fusion protein served as the adapter protein, and the N-terminal Aga2p bound Aga1p, which was immobilized on the surface of the cell. Aga2p bound specifically to Aga1p through disulfide bonds.

The three component system consisting of the Fn10-NorpA fusion protein, the Aga2p-InaD fusion protein, and the Aga1p cell surface protein were cloned into pPIC expression vectors, under the control of an inducible promoter. The inducible promoter used was the AOX1 promoter, which is induced by methanol. Thus when methanol was added to yeast cells transformed with the vectors, the three proteins were expressed. Aga1p was expressed on the surface of the cell. Aga2p-InaD was localized to the cell surface where the N-terminal region of Aga2p-InaD bound to Aga1p. Fn10-NorpA was localized to the secretory pathway, was secreted from the cell, and bound InaD via the C-terminal NorpA ligand (Figure 1). The system can also be switched such that the InaD is fused to Aga1 and NorpA is fused to Aga2p (See Figs. 12 and 13).

A c-myc epitope tag was fused between the NorpA ligand and the C-terminus of Fn10. The c-myc epitope allowed for the detection of the displayed fibronectin by using a c-myc antibody. The fluorescently labeled c-myc antibody bound to the c-myc epitope on the surface of the cell was detected by fluorescence-activated cell sorting (FACS).

### Strains and Media

The *Escherichia coli* Top10 strain (Invitrogen Carlsbad, CA) was used as the host strain for recombinant DNA manipulation. The *P. pastoris* GS 115 strain (Invitrogen Co., Carlsbad, CA) was used for the production of the fusion protein AGA2-InaD and HSA/MFalpha1-Fn10-NorpA. *E. coli* was cultivated in LB medium (1% tryptone, 0.5% yeast extract, and 0.5% sodium chloride) containing 100 µg/mL ampicillin. *P. pastoris* was cultivated in BMGY medium (1% yeast extract, 2% peptone, 100 mM potassium phosphate buffer (pH 6.0), 1.34% yeast nitrogen base, 4x10⁻⁵% biotin, and 1% glycerol), and BMMY medium (1% yeast extract, 2% peptone, 100 mM potassium phosphate buffer (pH 6.0), 1.34% yeast nitrogen base, 4x10⁻⁵% biotin, and 0.5-2.0% methanol).

### Construction of Expression Plasmids

The gene corresponding to AGA 1 was synthesized by Geneart and subcloned into pPIC3.5 (Invitrogen). The resulting vector was named pPIC3.5-AGA1 (Figure 2). The AGA2-InaD anchor gene was synthesized by Geneart (Germany) and subcloned into expression vector pPIC6a (Invitrogen) using BstI and EcoRI restriction sites. The resulting vector was named pPIC6-AGA2-InaD (Figure 4). The fibronectin construct consists of the MFalpha1/HSA hybrid leader followed by the fibronectin fused at the C-terminus to the NorpA ligand sequence. The complete gene was synthesized by Geneart (Germany) and subcloned into pPICHOLI-1 (Mobitec). The resulting vector was named pPICHOLI-1 MFalpha1Hsa-Fn10-NorpA (Figure 6).

### Yeast Transformation.

Electro-competent *P. pastoris* GS 115 (Invitrogen) strain was prepared according to the protocol specified by the supplier and co-transformed with SalI-digested pPIC3.5-AGA1, pPIC6-AGA2-InaD, and pPICHOLI-1 MFalpha1Hsa-Fn10-NorpA.

### Cultivation Conditions

The yeast transformants were precultivated in BMGY medium containing 100 ug/ml Zeocin and 200 ug/ml Blasticidin at 30°C for 16 hr. and used to inoculate 200 ml of BMGY medium (containing 100 ug/ml Zeocin and 200 ug/ml Balsticidin) in a 1 1 baffle flask to give an initial OD₆₀₀ value of 0.1. After 24 hr. of cultivation, the culture was centrifuged at 1000g for 10 min. and resuspended in BMMY medium (+100 ug/ml Zeocin and 200 ug/ml Blasticidin) containing 0.5%, 1.0%, or 2.0% methanol. To maintain the induction of the fusion proteins, 100% methanol was added every 24 hr. to the culture to the final concentrations mentioned above. Analysis of displayed fibronectins on the surface of yeast is performed using FACS and anti-myc antibody.

### Example 2: Switch system to secrete or display fibronectins on the surface of Pichia pastoris

One variant of the above display system enables the choice between secretion and display of proteins from *P. pastoris.* To achieve this, the fibronectin construct consisting of the MFalpha1/HSA hybrid leader followed by fibronectin fused at the C-terminus to the NorpA ligand sequence is cloned into pPICHOLI-C instead of pPICHOLI-1. The resulting vector is named pPICHOLI-C Mfalpha1Hsa-Fn10-NorpA (Figure 8). The key difference between the two vectors is the promoter, which in pPICHOLI-1 is the AOX1 promoter induced by methanol, and in the pPICHOLI-C is the Cup 1 promoter induced by copper. To display the fibronectin on the surface of *P. pastoris,* AGA1 and AGA2-InaD are induced with methanol, while pPICHOLI-C is induced with copper. This allows for the capture of the secreted fibronectin on the surface of yeast mediated through the tight InaD/NorpA interaction. For secretion of the fibronectin without displaying the protein on the surface of yeast, induction with copper is sufficient. Without the induction of AGA1 and AGA2-InaD (driven by methanol) the binding partner for NorpA (AGA1/AGA2-InaD) is not present on the surface of yeast and therefore the fibronectin will be secreted.

### Example 3: Yeast display using InaD/NorpA interaction in Saccharomyces cerevisiae

This example describes using the InaD/NorpA system with other yeast strains such as *Saccharomyces cerevisiae*

### Strains and Media

*Escherichia coli* Top10 (Invitrogen, Carlsbad, CA) was used as the host strain for recombinant DNA manipulation. The *S*. *cerevisiae* strain EBY100 (Invitrogen Co., Carlsbad, CA) was used for the production of the fusion proteins AGA2-InaD and MSalpha1/HSA-Fn10-NorpA or pYS6CT*MFalpha1-HSA-NorpA. *E. coli* was cultivated in LB medium (1% tryptone, 0.5% yeast extract, and 0.5% sodium chloride) containing 100u/g/mL ampicillin or 100 ug/ml Blasticidin. EBY100 was cultivated in CM medium-URA.

### Construction of Expression Plasmids

The InaD anchor gene was synthesized by Geneart (Germany) and subcloned in frame with the AGA2 anchor protein into the expression vector pYD NBC 1 (derivative of pYD1Invitrogen) using HindIII and EcoRI restriction sites. The resulting vector was named pYD_NBC1 AGA2-InaD (Figure 10). The fibronectin construct consists of the MFalphaI/HSA hybrid leader sequence followed by the fibronectin fused at its C-terminus to the NorpA ligand. The complete gene was synthesized by Geneart (Germany) and subcloned into pYS6CT (Invitrogen)), in which the origin of replication had been replaced by the CEN6/ARS4 region. The resulting vector was named pYS6CT_HSA_MFalpha1_Fn10_NorpA (Figure 12).

Plasmids were isolated from *E. coli* and the sequence confirmed. The purified plasmids were then co-transformed into EBY100 and plated out on selective media consisting of CM-TRP, + 200 ug/ml Blasticidin. Transformed colonies appeared within 2 days and were tested for display of Fibronectin by FACS analysis using an anti-myc antibody (ccccc).

### Example 4: Yeast display using Flo1-InaD/NorpA in Pichia pastoris

This example describes the use of an alternative expression system, Flo1, which is used with InaD/NorpA in *Pichia pastoris.*

### Strains and Media

The *Escherichia coli* Top10 strain (Invitrogen Carlsbad, CA) is used as the host strain for recombinant DNA manipulation. The *P. pastoris* GS 115 strain (Invitrogen Co., Carlsbad, CA) is used for the production of the fusion protein Flo1-InaD and HSA/MFalpha1-Fn10-NorpA. *E. coli* was cultivated in LB medium (1% tryptone, 0.5% yeast extract, and 0.5% sodium chloride) containing 100 *u*g/mL ampicillin. *P. pastoris* was cultivated in BMGY medium (1% yeast extract, 2% peptone, 100 mM potassium phosphate buffer (pH 6.0), 1.34% yeast nitrogen base, 4x10⁻⁵% biotin, and 1% glycerol), and BMMY medium (1% yeast extract, 2% peptone, 100 mM potassium phosphate buffer (pH 6.0), 1.34% yeast nitrogen base, 4x10⁻⁵% biotin, and 0.5-2.0% methanol).

### Construction of Expression Plasmids

The gene for Flo1, fused at the C-terminus to the PDZ domain of InaD, is synthesized by Geneart (Germany) and cloned into pPIC3.5 (Invitrogen) using a 5' EcoR1 site and a 3'Not1 site. The resulting plasmid is named pPIC3.5-Flo1-InaD. Expression of the fused protein is driven by the methanol inducible promoter AOX1. The fibronectin construct consists of the MFalpha1/HSA hybrid leader followed by the fibronectin fused at the C-terminus to the NorpA ligand sequence. The complete gene is synthesized by Geneart (Germany) and subcloned into pPICHOLI-1 (Mobitec). The resulting vector is named pPICHOLI-1 MFalpha1Hsa-Fn10-NorpA. Expression of the fibronectin construct is driven by the methanol inducible promoter AOX1.

### Yeast Transformation.

Electro-competent *P. pastoris* GS 115 (Invitrogen) strain is prepared according to the protocol specified by the supplier and co-transformed with Safl-digested pPIC3.5-FloI-InaD, and pPICHOLI-1 MFalpha1Hsa-Fn10-NorpA.

### Cultivation Conditions

The yeast transformants are precultivated in BMGY medium containing 100 ug/ml Zeocin and 200 ug/ml Blasticidin at 30°C for 16 hr. and used to inoculate 200 ml of BMGY medium (containing 100 ug/ml Zeocin and 200 ug/ml Balsticidin) in a 1 1 baffle flask to give an initial OD₆₀₀ value of 0.1. After 24 hr. of cultivation, the culture is centrifuged at 1000g for 10 min. and resuspended in BMMY medium (+100 ug/ml Zeocin and 200 ug/ml Blasticidin) containing 0.5%, 1.0%, or 2.0% methanol. To maintain the induction of the fusion proteins, 100% methanol is added every 24 hr. to the culture to the final concentrations mentioned above. Analysis of displayed fibronectins on the surface of yeast is performed using FACS and anti-myc antibody.

### Example 5: Screening of a fibronectin library

### Fibronectin Library Display

A fibronectin library is generated by methods well known in the art, including the method disclosed in US Patent No. 6,673,901. Other methods, such as use of error prone PCR, use of random priming techniques, or use of computational techniques are well known in the art and can also be used. The fibronectin library is designed with appropriate restriction enzyme cleavage sites in order to clone the library into yeast expression vectors.

The fibronectin library is displayed on a plurality of *P. pastoris* cells as described above in Example 1. The fibronectin library is modified to contain an MFalpha/HSA hybrid leader sequence fused to the N-terminus and a NorpA ligand sequence fused to the C-terminus. The modified fibronectin library is then cloned into the pPICHOLI-1 vector. As in the examples above, the expression of the fibronectin library is under the control of the AOX1 promoter. *P. pastoris* cells are transformed with the pPICHOLI-1 vectors expressing the fibronectin library and the vectors expressing Aga1p and Aga2p-InaD. Expression of the components is induced by the addition of methanol to the cells, and. the fibronectin library is displayed on a plurality of *P*. *pastoris* cells.

### Screening of Display Library

The yeast display fibronectin library is screened for binding to a target protein of interest using one of many methods known in the art. For example, the target protein is contacted with the yeast display fibronectin library under conditions that allow for the specific binding of the target protein to any members of the library. All bound target protein is now immobilized on the surface of a yeast cell. All unbound target protein is washed off. The bound target protein is fluorescently labeled by methods well known in the art, such as fluorescently labeled antibodies specific for the target protein. The labeled target protein, now immobilized on the surface of a yeast cell, is then detected using flow cytometry, i.e. FACS. Yeast cells that bind the labeled target protein will fluoresce and are sorted from those yeast cells that do not bind the target protein. The sorted yeast cells that have bound the target protein are clonally expanded , and the clone line or line containing members of the fibronectin library that bind the target protein are determined.

### Example 6: Screening of a protein library

### Protein Library Display

A protein library is generated by methods well known in the art, such as use of error prone PCR, use of random priming techniques, or use of computational techniques. The protein library is designed with appropriate restriction enzyme cleavage sites in order to clone the library into yeast expression vectors.

The cloned protein library is displayed on a plurality of *P. pastoris* cells as described above in Example 1. The protein library is modified to contain an MFalpha/HSA hybrid leader sequence fused to the N-terminus and a NorpA ligand sequence fused to the C-terminus. The modified protein library is then cloned into the pPICHOLI-1 vector. As in the examples above, the expression of the protein library is under the control of the AOX1 promoter. *P. pastoris* cells are transformed with the pPICHOLI-1 vectors expressing the protein library and the vectors expressing Aga1p and Aga2p-InaD. Expression of the components is induced by the addition of methanol to the cells, and. the protein library is displayed on a plurality of *P. pastoris* cells.

### Screening of Display Library

The yeast display protein library is screened for binding to a target protein of interest using one of many methods known in the art. For example, the target protein is contacted with the yeast display protein library under conditions that allow for the specific binding of the target protein to any members of the library. All bound target protein is now immobilized on the surface of a yeast cell. All unbound target protein is washed off. The bound target protein is fluorescently labeled by methods well known in the art, such as fluorescently labeled antibodies specific for the target protein. The labeled target protein, now immobilized on the surface of a yeast cell, is then detected using flow cytometry, i.e. FACS. Yeast cells that bind the labeled target protein will fluoresce and are sorted from those yeast cells that do not bind the target protein. The sorted yeast cells that have bound the target protein are clonally expanded , and the clone line or line containing members of the protein library that bind the target protein are determined.

### Example 7: Screening of a Fibronectin or HSA libraries

### Fibronectin or HSA Library Display

A fibronectin or HSA library is generated by methods well known in the art, such as use of error prone PCR, use of random priming techniques, or use of computational techniques. The libraries are designed with appropriate restriction enzyme cleavage sites in order to clone the libraries into yeast expression vectors (See Fig. 8 and SEQ ID NO: 10).

The cloned fibronectin library or HSA library is displayed on a plurality of *P*. *pastoris* cells as described above in Example 1. The fibronectin library is modified to contain an MFalpha/HSA hybrid leader sequence fused to the N-terminus and a NorpA ligand sequence fused to the C-terminus (pYS HSA_MFalphal Fn10 NorpA). The HSA library is modified to contain a MFalpha CT (C-Terminal) It is a left-over of the original Invitrogen vector used for the constructions. If you look at the vector map (e.g. figure 13) you can see a c-terminal v5 and 6xhis sequence of the c-terminus of the insert. I've placed a stop in front of it and it is not translated in the final displayed protein. leader sequence (pYS6/CT HSA-NorpA). The modified fibronectin or HSA library is then cloned into the pYS vector. The expression of the fibronectin or HSA library is under the control of the T7 promoter. *P. pastoris* cells are transformed with the pYS vectors expressing the fibronectin or HSA library and the vectors expressing Aga1p and Aga2p-InaD. Expression of the components is induced by the addition of methanol to the cells, and. the fibronectin or HSA library is displayed on a plurality of *P. pastoris* cells.

### (a) FACS analysis of protein surface expression.

Yeast cells expressing either fibronectin (pYS HSA_MFalpha1 Fn10 NorpA) or HSA (pYS6/CT HSA-NorpA) were stained with anti-myc antibody, followed by APC labeled secondary anti-mouse antibody and then subjected to FACS analysis. The results of the analysis are shown in Figs. 9A-E. Specifically, Fig 9A is the control sample showing unstained yeast cells; Fig. 9B is a sample of uninduced yeast cells expressing fibronectin; Fig. 9C is a sample of induced yeast cells expressing fibronectin, showing a shift of cells compared with the uninduced cells; Fig. 9D is a sample of uninduced yeast cells expressing HSA; and Fig. 9E is a sample of induced yeast cells expressing HSA, again showing a shift of cells compared with the uninduced cells. These results clearly demonstrate that the yeast display system is able to express fibronectin molecules, and proteins, such as HSA.

### (b) Fluorometric mircovolume assay technology (FMAT, Perkin Elmer)analysis of yeast expressing fibronectin or HSA

Yeast cells expressing either fibronectin (plasmid) or HSA (plasmid) were also analyzed by FMAT by staining with anti-myc antibody and APC labeled secondary anti-mouse antibody. The samples were then subjected to FMAT confocal fluorescence microscopy and shown in Figs. 10A-E. Those colonies that express fibronectin or HSA appear as white dots against a black background. Specifically, Fig 10A is the control sample showing unstained yeast cells and appears entirely black; Fig. 10B is a sample of uninduced yeast cells expressing fibronectin. The uninduced yeast cells do not produce fibronectin and are not detected (image appears black); Fig. 10C is a sample of induced yeast cells expressing fibronectin. In this instance, induction leads to the fibronectin with a myc tag being expressed and detected using the anti-myc antibody. Subsequent detection with the APC secondary anti-mouse antibody and FMAT confocal fluorescence microscopy results in visible white colonies being detected. Fig.10D is a sample of uninduced yeast cells expressing HSA, as before the uninduced yeast cells do not produce fibronectin and the image appears black; and Fig. 10E is a sample of induced yeast cells expressing HSA, again showing small white colonies compared with the uninduced cells. These results further confirm that the yeast display system is able to express fibronectin molecules, and proteins, such as HSA.

### Example 8: Screening of single chain Fv libraries

### Single Chain Fv Library Display

A single chain Fv library is generated by methods well known in the art, such as use of error prone PCR, use of random priming techniques, or use of computational techniques. The libraries are designed with appropriate restriction enzyme cleavage sites in order to clone the libraries into yeast expression vectors (See Fig. 11 and SEQ ID NO: 11).

The cloned scFv lysozyme library is displayed on a plurality of *P. pastoris* cells as described above in Example 1. The scFv library is modified to contain an MFalpha leader sequence fused to the N-terminus and a NorpA ligand sequence fused to the C-terminus (pYS6/CT* MFalpha1-scFv lysozyme-NorpA). The modified scFv lysozyme library is then cloned into the pYS vector. The expression of the scFv lysozyme is under the control of the T7 promoter. *P. pastoris* cells are transformed with the pYS vectors expressing the scFv lysozyme library and the vectors expressing Aga1p and Aga2p-InaD. Expression of the components is induced by the addition of methanol to the cells, and the fibronectin or HSA library is displayed on a plurality of *P. pastoris* cells.

### (a) FACS analysis of protein surface expression.

Yeast cells expressing the scFv lysozyme were stained with anti-myc antibody, followed by APC labeled secondary anti-mouse antibody and then subjected to FACS analysis.

### (b) FMAT analysis of yeast expressing fibronectin or HSA

Yeast cells expressing the scFv lysozyme were also analyzed by FMAT by staining with anti-myc antibody and APC labeled secondary anti-mouse antibody. The samples were then subjected to FMAT confocal fluorescence microscopy.

### Example 9: Screening of protein libraries with a reverse system

### Protein Library Display

In the Example, the yeast display system described herein is reversed such that NorpA is fused to Aga2 and the InaD is fused to Agal. A protein library is generated by methods well known in the art, such as use of error prone PCR, use of random priming techniques, or use of computational techniques. The libraries are designed with appropriate restriction enzyme cleavage sites in order to clone the libraries into yeast expression vectors (See Figs 12 and 13 and SEQ ID NOs: 12 and 13).

The protein display system for *P. pastoris* was developed to display a fibronectin type III domain (Fn10), by fusing a leader sequence (MFalpha1) at N-terminus of InaD and fusing the Fn10 to its C-terminus. Once expressed, the Fn10 was secreted from the cell and the PDZ domain of InaD bound to the NorpA ligand through disulfide bonds. The NorpA was fused to the C-terminus of the Agap2 protein. The Aga2p-NorpA fusion protein served as the adapter protein, and the N-terminal Aga2p bound Aga1p, which was immobilized on the surface of the cell. Aga2p bound specifically to Aga1p through disulfide bonds.

The three component system consisting of the Aga2p-NorpA fusion protein, the Aga1-InaD fusion protein, and the Aga1p cell surface protein were cloned into pPD and pYS expression vectors respectively, under the control of a Gal inducible promoter.

The inducible promoter used was the Gal 1 promoter, which is induced by galactose. Thus when galactose was added to yeast cells transformed with the vectors, the three proteins were expressed. Aga1p was expressed on the surface of the cell. Aga2p-Norp was localized to the cell surface where the N-terminal region of Aga2p-NorpA bound to Aga1p. Fn100-InaD was localized to the secretory pathway, was secreted from the cell, and bound NorpA via the C-terminal InaD ligand.

### SEQUENCES

pPIC3.5 AGA 1 (956bp - 3136bp, direct) 242aa

MTLSFAHFTY LFTILLGLTN IALASDPETI LVTITKTNDA NGVVTTTVSP
ALVSTSTIVQ AGTTTLYTTW CPLTVSTSSA AEISPSISYA TTLSRFSTLT
LSTEVCSHEA CPSSSTLPTT TLSVTSKFTS YICPTCHTTA ISSLSEVGTT
TVVSSSAIEP SSASIISPVT STLSSTTSSN PTTTSLSSTS TSPSSTSTSP
SSTSTSSSST STSSSSTSTS SSSTSTSPSS TSTSSSLTST SSSSTSTSQS
STSTSSSSTS TSPSSTSTSS SSTSTSPSSK STSASSTSTS SYSTSTSPSL
TSSSPTLAST SPSSTSISST FTDSTSSLGS SIASSSTSVS LYSPSTPVYS
VPSTSSNVAT PSMTSSTVET TVSSQSSSEY ITKSSISTTI PSFSMSTYFT
TVSGVTTMYT TWCPYSSESE TSTLTSMHET VTTDATVCTH ESCMPSQTTS
LITSSIKMST KNVATSVSTS TVESSYACST CAETSHSYSS VQTASSSSVT
QQTTSTKSWV SSMTTSDEDF NKHATGKYHV TSSGTSTIST SVSEATSTSS
IDSESQEQSS HLLSTSVLSS SSLSATLSSD STILLFSSVS SLSVEQSPVT
TLQISSTSEI LQPTSSTAIA TISASTSSLS ATSISTPSTS VESTIESSSL
TPTVSSIFLS SSSAPSSLQT SVTTTEVSTT SISIQYQTSS MVTISQYMGS
GSQTRLPLGK LVFAIMAVAC NVIFS (SEQ ID NO: 2)

pPIC6 A AGA2-InaD (941bp - 1648bp, direct) 78aa

MQLLRCFSIF SVIASVLAQE LTTICEQIPS PTLESTPYSL STTTILANGK
AMQGVFEYYK SVTFVSNCGS HPSTTSKGSP INTQYVFKLL QASGGGGSGG
GGSGGGGSAS MTGGQQMGRE NLYFQGVPGS SVVSRAGELI HMVTLDKTGK
KSFGICIVRG EVKDSPNTKT TGIFIKGIVP DSPAHLCGRL KVGDRILSLN
GKDVRNSTEQ AVIDLIKEAD FKIELEIQTF DK (SEQ ID NO: 3)

pPICHOLI-1_MFalpha1Hsa-Fn10-NorpA (884bp - 1441bp, direct) 62aa

MKWVSFISLL FLFSSAYSRS LDKRENLYFQ GGSVSDVPRD LEVVAATPTS
LLISWDAPAV TVRYYRITYG ETGGNSPVQE FTVPGSKSTA TISGLKPGVD
YTITVYAVTG RGDSPASSKP ISINYRTEFE NLYFQGSGGG GEQKLISEED
LHHHHHHPST PPTPSPSTPP TPSPSYKTQG KTEFCA (SEQ ID NO: 4)

pPICHOLI-C Mfalpha1Hsa-Fn10-NorpA (691bp - 1248bp, direct) 62aa

MKWVSFISLL FLFSSAYSRS LDKRENLYFQ GGSVSDVPRD LEVVAATPTS
LLISWDAPAV TVRYYRITYG ETGGNSPVQE FTVPGSKSTA TISGLKPGVD
YTITVYAVTG RGDSPASSKP ISINYRTEFE NLYFQGSGGG GEQKLISEED
LHHHHHHPST PPTPSPSTPP TPSPSYKTQG KTEFCA (SEQ ID NO: 5)

pYD_NBC1_Aga2-InaD (534bp - 1235bp, direct) 78aa

MQLLRCFSIF SVIASVLAQE LTTICEQIPS PTLESTPYSL STTTILANGK
AMQGVFEYYK SVTFVSNCGS HPSTTSKGSP INTQYVFKLL QASGGGGSGG
GGSGGGGSAS MTGGQQMGRE NLYFQGVPGS SVVSRAGELI HMVTLDKTGK
KSFGICIVRG EVKDSPNTKT TGIFIKGIVP DSPAHLCGRL KVGDRILSLN
GKDVRNSTEQ AVIDLIKEAD FKIELEIQTF DK (SEQ ID NO: 6)

pYS6/CT_HSA_MFa1pha1_Fn10_NorpA (513bp - 1080bp, direct) 63aa

MKWVSFISLL FLFSSAYSRS LDKRENLYFQ GGSVSDVPRD LEVVAATPTS
LLISWDAPAV TVRYYRITYG ETGGNSPVQE FTVPGSKSTA TISGLKPGVD
YTITVYAVTG RGDSPASSKP ISINYRTEFE NLYFQGSGGG GEQKLISEED
LHHHHHHPST PPTPSPSTPP TPSPSYKTQG KTEFCA (SEQ ID NO: 7)

InaD PDZ domain amino acid sequence (InaD aa 11-107)

AGELIHMVTL DKTGKKSFGI CIVRGEVKDS PNTKTTGIFI KGIVPDSPAH
LCGRLKVGDR ILSLNGKDVR NSTEQAVIDL IKEADFKIEL EIQTFDK (SEQ ID
NO: 8)

NorpA C-terminal 11 amino acids including EFCA motif
YKTQGKTEFC A (SEQ ID NO: 9)

pYS6/CT* MFalpha HSA-NorpA

MRFPSIFTAVLFAASSALAAPVNTTTEDETAQIPAEAVIGYSDLEGDFDVAVLPFSNST
NNGLLFINTTIASIAAKEEGVSLEKREAEAASDAHKSEVAHRFKDLGEENFKALVLIAF
AQYLQQCPFEDHVKLVNEVTEFAKTCVADESAENCDKSLHTLFGDKLCTVATLRETYGE
MADCCAKQEPERNECFLQHKDDNPNLPRLVRPEVDVMCTAFHDNEETFLKKYLYEIARR
HPYFYAPELLFFAKRYKAAFTECCQAADKAACLLPKLDELRDEGKASSAKQRLKCASLQ
KFGERAFKAWAVARLSQRFPKAEFAEVSKLVTDLTKVHTECCHGDLLECADDRADLAKY
ICENQDSISSKLKECCEKPLLEKSHCIAEVENDEMPADLPSLAADFVESKDVCKNYAEA
KDVFLGMFLYEYARRHPDYSVVLLLRLAKTYETTLEKCCAAADPHECYAKVFDEFKPLV
EEPQNLIKQNCELFEQLGEYKFQNALLVRYTKKVPQVSTPTLVEVSRNLGKVGSKCCKH
PEAKRMPCAEDYLSVVLNQLCVLHEKTPVSDRVTKCCTESLVNRRPCFSALEVDETYVP
KEFNAETFTFHADICTLSEKERQIKKQTALVELVKHKPKATKEQLKAVMDDFAAFVEKC
CKADDKETCFAEEGKKLVAASQAALGLGSENLYFQGSGGGGEQKLISEEDLHHHHHHHH
PSTPPTPSPSTPPTPSPSYKTQGKTEFCA (SEQ ID NO: 10).

pYS6/CT* MFa1pha1-scFv lysozyme-NorpA

MRFPSIFTAVLFAASSALAAPVNTTTEDETAQIPAEAVIGYSDLEGDFDVAVLPFSNS
TNNGLLFINTTIASIAAKEEGVSLEKREAEAASQVKLQQSGAELVKPGASVKLSCTASG
FNIKDTYMHWVKQRPEQGLEWIGRIDPANGNTKYDPKFQGKATITADTSSNTAYLQLSS
LTSEDTAVYYCARWDWYFDVWGQGTTVTVSSGGGGSGGGGSGGGGSDIELTQSPSSMYT
SLGERVTITCKASQDINSYLRWFQQKPGKSPKTLIYYATSLADGVPSRFSGSGSGQDYS
LTISSLESDDTTTYYCLQHGESPYTFGGGTKLEIKRAAAEQKLISEEDLNGSENLYFQG
SGGGGEQKLISEEDLHHHHHHHHPSTPPTPSPSTPPTPSPSYKTQGKTEFCA (SEQ
ID NO: 11)

pYD_NBC1_Aga2-NorpA

MQLLRCFSIFSVIASVLAQELTTICEQIPSPTLESTPYSLSTTTILANGKAMQGVFEYY
KSVTFVSNCGSHPSTTSKGSPINTQYVFKLLQASGGGGSGGGGSYKTQGKTEFCA
(SEQ ID NO: 12)

pYS6/CT* MFa1pha1-InaD-Fn10 (507bp - 1487bp, direct) 109aa

MRFPSIFTAVLFAASSALAAPVNTTTEDETAQIPAEAVIGYSDLEGDFDVAVLPFSNST
NNGLLFINTTIASIAAKEEGVSLEKREAEAASAGELIHMVTLDKTGKKSFGICIVRGEV
KDSPNTKTTGIFIKGIVPDSPAHLCGRLKVGDRILSLNGKDVRNSTEQAVIDLIKEADF
KIELEIQTFDKSGGGGEQKLISEEDLHHHHHHPSTPPTPSPSTPPTPSPENLYFQGVSD
VPRDLEVVAATPTSLLISWDAPAVTVRYYRITYGETGGNSPVQEFTVPGSKSTATISGL
KPGVDYTITVYAVTGRGDSPASSKPISINYRT (SEQ ID NO: 13)

## Claims

1. A library of host cells, wherein each host cell comprising:
(a) a cell surface molecule attached to the surface of the cell,
(b) an adapter molecule comprising a first binding site and a second binding site, and
(c) a display molecule comprising a modified polypeptide, wherein the modified polypeptide is selected from the group consisting of: a scaffold protein, a signal transduction protein, an antibody, an immunoglobulin, an immunoadhesin, a receptor, a ligand, an oncoprotein, a transcription factor, an enzyme, and a fibronectin polypeptide;
wherein the first binding site binds specifically to the cell surface molecule and cannot bind to the display molecule and the second binding site binds specifically to the display molecule and cannot bind to the cell surface molecule,
wherein the adapter molecule is not a component of the modified polypeptide, and wherein each host cell comprises a different modified polypeptide.

2. The library of host cells of claim 1 wherein the host cells of said library comprising a plurality of display molecules.

3. The library of host cells of claim 1 or claim 2, wherein the host cell surface molecule is covalently linked to the first binding site.

4. The library of host cells of any of claims 1-3, wherein the host cell surface molecule comprises a first agglutinin that is Agalp, and wherein the first binding site comprises a second agglutinin that is Aga2p.

5. The library of host cells of any of claims 1-4, wherein the host cell surface molecule is attached to the cell membrane via a GPI anchor.

6. The library of host cells of any of claims 1-5, wherein the second binding site is covalently linked to the display molecule.

7. The library of host cells of any of claims 1-6, wherein the second binding site comprises a PDZ domain of InaD.

8. The library of host cells of claim 7, wherein the display molecule comprises a C-terminal NorpA ligand.

9. The library of host cells of any of claims 1-6, wherein the display molecule comprises a PDZ domain of InaD.

10. The library of host cells of claim 9, wherein the second binding site comprises a C-terminal NorpA ligand.

11. The library of host cells of any of claims 1-10, wherein the fibronectin polypeptide comprises an F10 polypeptide.

12. The library of host cells of any of claims 1-11, wherein the display molecule comprises a secretion signal peptide.

13. The library of host cells of claim 12, wherein the secretion signal peptide comprises an MFalpha/HSA hybrid leader peptide, or an MFalpha leader peptide.

14. The library of host cells of any of claims 1-13, wherein expression of the display molecule is under the control of a first inducible promoter selected from the group consisting of an AOX1 promoter, a Cup 1 promoter, and a Gal 1 promoter.

15. The library of host cells of any of claims 1-14, wherein the expression of the adapter molecule is under the control of a second inducible promoter selected from the group consisting of an AOX1 promoter, a Cup 1 promoter, and a Gal 1 promoter.

16. The library of host cells of any of claims 1-15, wherein the host cell is a yeast cell.

17. A method for displaying a modified polypeptide comprising;
(a) providing a host cell comprising a cell surface molecule attached to the surface of the cell which is expressed from an expression vector in the host cell and a first nucleic acid encoding a display polypeptide comprising a modified polypeptide, wherein the modified polypeptide is selected from the group consisting of: a scaffold protein, a signal transduction protein, an antibody, an immunoglobulin, an immunoadhesin, a receptor, a ligand, an oncoprotein, a transcription factor, an enzyme, and a fibronectin polypeptide,
(b) contacting the host cell with an adapter molecule comprising a first binding site and a second binding site under conditions wherein the first binding site binds to the cell surface molecule, and
(c) incubating the host cell under conditions wherein the host cell exports the display polypeptide outside the host cell under conditions wherein the second binding site binds to the display polypeptide,
wherein the first binding site binds specifically to the cell surface molecule and cannot bind to the display molecule and the second binding site binds specifically to the display polypeptide and cannot bind to the cell surface molecule, and wherein the adapter molecule is not a component of the modified polypeptide.

18. A method for generating a host cell display library comprising:
introducing into a plurality of host cells a display library of first nucleic acids each encoding a display polypeptide comprising a modified polypeptide, wherein the modified polypeptide is selected from the group consisting of: a scaffold protein, a signal transduction protein, an antibody, an immunoglobulin, an immunoadhesin, a receptor, a ligand, an oncoprotein, a transcription factor, an enzyme, and a fibronectin polypeptide, wherein at least two of the introduced first nucleic acids encode different modified polypeptides,
wherein each host cell comprises a second nucleic acid which encodes a cell surface polypeptide and a third nucleic acid which encodes an adapter molecule comprising a first binding site and a second binding site, wherein the first binding site binds to the cell surface molecule but not the display polypeptide and the second binding site binds to the display polypeptide but not the cell surface molecule, and wherein the adapter molecule is not a component of the modified polypeptide.

## Patentansprüche

1. Eine Wirtszellenbibliothek, wobei jede Wirtszelle Folgendes umfasst:
(a) ein Zelloberflächenmolekül, angebracht an der Oberfläche der Zelle,
(b) ein Adaptermolekül, umfassend eine erste Bindungsstelle und eine zweite Bindungsstelle, und
(c) ein Anzeigemolekül, umfassend ein modifiziertes Polypeptid, wobei das modifizierte Polypeptid ausgewählt ist aus der Gruppe bestehend aus: einem Gerüstprotein, einem Signaltransduktionsprotein, einem Antikörper, einem Immunglobulin, einem Immunadhäsin, einem Rezeptor, einem Liganden, einem Onkoprotein, einem Transkriptionsfaktor, einem Enzym und einem Fibronectin-Polypeptid;
wobei die erste Bindungsstelle an das Zelloberflächenmolekül spezifisch bindet und nicht an das Anzeige-Polypeptid binden kann und die zweite Bindungsstelle an das Anzeige-Polypeptid spezifisch bindet und nicht an das Zelloberflächenmolekül binden kann,
wobei das Adaptermolekül keine Komponente des modifizierten Polypeptids ist und wobei jede Wirtszelle ein unterschiedliches modifiziertes Polypeptid umfasst.

2. Die Wirtszellenbibliothek nach Anspruch 1, wobei die Wirtszellen dieser Bibliothek eine Vielzahl von Anzeigemolekülen umfassen.

3. Die Wirtszellenbibliothek nach Anspruch 1 oder Anspruch 2, wobei das Wirtszellenoberflächenmolekül kovalent an die erste Bindungsstelle gebunden ist.

4. Die Wirtszellenbibliothek nach einem der Ansprüche 1-3, wobei das Wirtszellenoberflächenmolekül ein erstes Agglutinin umfasst, das Aga1p ist, und wobei die erste Bindungsstelle ein zweites Agglutinin umfasst, das Aga2p ist.

5. Die Wirtszellenbibliothek nach einem der Ansprüche 1-4, wobei das Wirtszellenoberflächenmolekül an der Zellmembran über einen GPI-Anker angebracht ist.

6. Die Wirtszellenbibliothek nach einem der Ansprüche 1-5, wobei die zweite Bindungsstelle kovalent an das Anzeigemolekül gebunden ist.

7. Die Wirtszellenbibliothek nach einem der Ansprüche 1-6, wobei die zweite Bindungsstelle eine PDZ-Domäne von InaD umfasst.

8. Die Wirtszellenbibliothek nach Anspruch 7, wobei das Anzeigemolekül einen C-terminalen NorpA-Liganden umfasst.

9. Die Wirtszellenbibliothek nach einem der Ansprüche 1-6, wobei das Anzeigemolekül eine PDZ-Domäne von InaD umfasst.

10. Die Wirtszellenbibliothek nach Anspruch 9, wobei die zweite Bindungsstelle einen C-terminalen NorpA-Liganden umfasst.

11. Die Wirtszellenbibliothek nach einem der Ansprüche 1-10, wobei das Fibronectin-Polypeptid ein F10-Polypeptid umfasst.

12. Die Wirtszellenbibliothek nach einem der Ansprüche 1-11, wobei das Anzeigemolekül ein Sekretionssignalpeptid umfasst.

13. Die Wirtszellenbibliothek nach Anspruch 12, wobei das Sekretionssignalpeptid ein MFalpha/HSA-Hybrid-Leitpeptid oder ein MFalpha-Leitpeptid umfasst.

14. Die Wirtszellenbibliothek nach einem der Ansprüche 1-13, wobei die Expression des Anzeigemoleküls unter der Steuerung eines ersten induzierbaren Promotors steht, ausgewählt aus der Gruppe bestehend aus einem AOX1-Promotor, einem Cup1-Promotor und einem Gall-Promotor.

15. Die Wirtszellenbibliothek nach einem der Ansprüche 1-14, wobei die Expression des Adaptermoleküls unter der Steuerung eines zweiten induzierbaren Promotors steht, ausgewählt aus der Gruppe bestehend aus einem AOX1-Promotor, einem Cup1-Promotor und einem Gall-Promotor.

16. Die Wirtszellenbibliothek nach einem der Ansprüche 1-15, wobei die Wirtszelle eine Hefezelle ist.

17. Ein Verfahren zum Anzeigen eines modifizierten Polypeptids, umfassend:
(a) Bereitstellen einer Wirtszelle, umfassend ein Zelloberflächenmolekül, das an der Oberfläche der Zelle angebracht ist und von einem Expressionsvektor in der Wirtszelle exprimiert wird, und eine erste Nukleinsäure, die ein Anzeige-Polypeptid codiert, das ein modifiziertes Polypeptid umfasst, wobei das modifizierte Polypeptid ausgewählt ist aus der Gruppe bestehend aus: einem Gerüstprotein, einem Signaltransduktionsprotein, einem Antikörper, einem Immunglobulin, einem Immunadhäsin, einem Rezeptor, einem Liganden, einem Onkoprotein, einem Transkriptionsfaktor, einem Enzym und einem Fibronectin-Polypeptid,
(b) Kontaktieren der Wirtszelle mit einem Adaptermolekül, umfassend eine erste Bindungsstelle und eine zweite Bindungsstelle, unter Bedingungen, wobei die erste Bindungsstelle an das Zelloberflächenmolekül bindet, und
(c) Inkubieren der Wirtszelle unter Bedingungen, wobei die Wirtszelle das Anzeige-Polypeptid aus der Wirtszelle unter Bedingungen exportiert, wobei die zweite Bindungsstelle an das Anzeige-Polypeptid bindet,
wobei die erste Bindungsstelle an das Zelloberflächenmolekül spezifisch bindet und nicht an das Anzeige-Polypeptid binden kann und die zweite Bindungsstelle an das Anzeige-Polypeptid spezifisch bindet und nicht an das Zelloberflächenmolekül binden kann und wobei das Adaptermolekül keine Komponente des modifizierten Polypeptids ist.

18. Ein Verfahren zum Erzeugen einer Wirtszellenanzeigebibliothek, umfassend:
Einbringen in eine Vielzahl von Wirtszellen einer Anzeigebibliothek von ersten Nukleinsäuren, wobei jede Nukleinsäure ein Anzeige-Polypeptid codiert, umfassend ein modifiziertes Polypeptid, wobei das modifizierte Polypeptid ausgewählt ist aus der Gruppe bestehend aus: einem Gerüstprotein, einem Signaltransduktionsprotein, einem Antikörper, einem Immunglobulin, einem Immunadhäsin, einem Rezeptor, einem Liganden, einem Onkoprotein, einem Transkriptionsfaktor, einem Enzym und einem Fibronectin-Polypeptid, wobei mindestens zwei der eingebrachten ersten Nukleinsäuren unterschiedliche, modifizierte Polypeptide codieren,
wobei jede Wirtszelle eine zweite Nukleinsäure, die ein Zelloberflächen-Polypeptid codiert, und eine dritte Nukleinsäure umfasst, die ein Adaptermolekül codiert, das eine erste Bindungsstelle und eine zweite Bindungsstelle umfasst, wobei die erste Bindungsstelle an das Zelloberflächenmolekül bindet, jedoch nicht an das Anzeige-Polypeptid, und die zweite Bindungsstelle an das Anzeige-Polypeptid bindet, jedoch nicht an das Zelloberflächenmolekül, und wobei das Adaptermolekül keine Komponente des modifizierten Polypeptids ist.

## Revendications

1. Banque de cellules hôtes, dans laquelle chaque cellule hôte comprenant :
(a) une molécule de surface cellulaire fixée à la surface de la cellule,
(b) une molécule adaptatrice comprenant un premier site de liaison et un second site de liaison et
(c) une molécule de présentation comprenant un polypeptide modifié, dans laquelle le polypeptide modifié est choisi dans le groupe constitué par : une protéine d'échafaudage, une protéine de transduction de signal, un anticorps, une immunoglobuline, une immuno-adhésine, un récepteur, un ligand, une oncoprotéine, un facteur de transcription, une enzyme et un polypeptide fibronectine ;
dans laquelle le premier site de liaison se lie spécifiquement à la molécule de surface cellulaire et ne peut pas se lier à la molécule de présentation et le second site de liaison se lie spécifiquement à la molécule de présentation et ne peut pas se lier à la molécule de surface cellulaire,
dans laquelle la molécule adaptatrice n'est pas un composant du polypeptide modifié
et dans laquelle chaque cellule hôte comprend un polypeptide modifié différent.

2. Banque de cellules hôtes de la revendication 1, dans laquelle les cellules hôtes de ladite banque comprenant une pluralité de molécules de présentation.

3. Banque de cellules hôtes de la revendication 1 ou la revendication 2, dans laquelle la molécule de surface de la cellule hôte est liée de façon covalente au premier site de liaison.

4. Banque de cellules hôtes de l'une quelconque des revendications 1-3, dans laquelle la molécule de surface de la cellule hôte comprend une première agglutinine qui est Agalp et dans laquelle le premier site de liaison comprend une seconde agglutinine qui est Aga2p.

5. Banque de cellules hôtes de l'une quelconque des revendications 1-4, dans laquelle la molécule de surface de la cellule hôte est fixée à la membrane cellulaire par l'intermédiaire d'une ancre GPI.

6. Banque de cellules hôtes de l'une quelconque des revendications 1-5, dans laquelle le second site de liaison est lié de façon covalente à la molécule de présentation.

7. Banque de cellules hôtes de l'une quelconque des revendications 1-6, dans laquelle le second site de liaison comprend un domaine PDZ d'InaD.

8. Banque de cellules hôtes de la revendication 7, dans laquelle la molécule de présentation comprend un ligand NorpA C-terminal.

9. Banque de cellules hôtes de l'une quelconque des revendications 1-6, dans laquelle la molécule de présentation comprend un domaine PDZ d'InaD.

10. Banque de cellules hôtes de la revendication 9, dans laquelle le second site de liaison comprend un ligand NorpA C-terminal.

11. Banque de cellules hôtes de l'une quelconque des revendications 1-10, dans laquelle le polypeptide fibronectine comprend un polypeptide F10.

12. Banque de cellules hôtes de l'une quelconque des revendications 1-11, dans laquelle la molécule de présentation comprend un peptide signal de sécrétion.

13. Banque de cellules hôtes de la revendication 12, dans laquelle le peptide signal de sécrétion comprend un peptide meneur hybride MFalpha/HSA ou un peptide meneur MFalpha.

14. Banque de cellules hôtes de l'une quelconque des revendications 1-13, dans laquelle l'expression de la molécule de présentation est sous le contrôle d'un premier promoteur inductible choisi dans le groupe constitué par un promoteur AOX1, un promoteur Cup 1 et un promoteur Gal 1.

15. Banque de cellules hôtes de l'une quelconque des revendications 1-14, dans laquelle l'expression de la molécule adaptatrice est sous le contrôle d'un second promoteur inductible choisi dans le groupe constitué par un promoteur AOX1, un promoteur Cup 1 et un promoteur Gal 1.

16. Banque de cellules hôtes de l'une quelconque des revendications 1-15, dans laquelle la cellule hôte est une cellule de levure.

17. Procédé de présentation d'un polypeptide modifié comprenant ;
(a) l'apport d'une cellule hôte comprenant une molécule de surface cellulaire fixée à la surface de la cellule, qui est exprimée à partir d'un vecteur d'expression dans la cellule hôte, et d'un premier acide nucléique codant pour un polypeptide de présentation comprenant un polypeptide modifié,
dans laquelle le polypeptide modifié est choisi dans le groupe constitué par : une protéine d'échafaudage, une protéine de transduction de signal, un anticorps, une immunoglobuline, une immuno-adhésine, un récepteur, un ligand, une oncoprotéine, un facteur de transcription, une enzyme et un polypeptide fibronectine,
(b) la mise en contact de la cellule hôte avec une molécule adaptatrice, comprenant un premier site de liaison et un second site de liaison, dans des conditions dans lesquelles le premier site de liaison se lie à la molécule de surface cellulaire et
(c) l'incubation de la cellule hôte dans des conditions dans lesquelles la cellule hôte exporte le polypeptide de présentation hors de la cellule hôte, dans des conditions dans lesquelles le second site de liaison se lie au polypeptide de présentation,
dans lequel le premier site de liaison se lie spécifiquement à la molécule de surface cellulaire et ne peut pas se lier à la molécule de présentation et le second site de liaison se lie spécifiquement au polypeptide de présentation et ne peut pas se lier à la molécule de surface cellulaire, et dans lequel la molécule adaptatrice n'est pas un composant du polypeptide modifié.

18. Procédé de génération d'une banque de présentation de cellules hôtes comprenant :
l'introduction, dans une pluralité de cellules hôtes, d'une banque de présentation de premiers acides nucléiques codant chacun pour un polypeptide de présentation comprenant un polypeptide modifié, dans laquelle le polypeptide modifié est choisi dans le groupe constitué par : une protéine d'échafaudage, une protéine de transduction de signal, un anticorps, une immunoglobuline, une immuno-adhésine, un récepteur, un ligand, une oncoprotéine, un facteur de transcription, une enzyme et un polypeptide fibronectine,
dans laquelle au moins deux des premiers acides nucléiques introduits codent pour différents polypeptides modifiés,
dans lequel chaque cellule hôte comprend un deuxième acide nucléique, qui code pour un polypeptide de surface cellulaire, et un troisième acide nucléique qui code pour une molécule adaptatrice comprenant un premier site de liaison et un second site de liaison, dans laquelle le premier site de liaison se lie à la molécule de surface cellulaire mais non au polypeptide de présentation et le second site de liaison se lie au polypeptide de présentation mais non à la molécule de surface cellulaire, et dans laquelle la molécule adaptatrice n'est pas un composant du polypeptide modifié.
